# EUROPÄISCHE PATENTSCHRIFT

(11) **EP 3 318 296 B1**
(45) Veröffentlichungstag und Bekanntmachung des Hinweises auf die Patenterteilung: **30.12.2020**
(21) Anmeldenummer: 17200296.6
(22) Anmeldetag: 07.11.2017
(51) Int. Cl.: A61M 16/20

(54) **EXPIRATIONSVENTIL**
EXPIRATION VALVE
SOUPAPE D'EXPIRATION

(30) Priorität: 08.11.2016 DE 102016121382
(43) Veröffentlichungstag der Anmeldung: 09.05.2018
(73) Patentinhaber: Hamilton Medical AG, 7402 Bonaduz (CH)
(72) Erfinder: Breitruck, Felix, 9470 Buchs SG (CH); De-Stefani, Dino, 7000 Chur (CH)
(74) Vertreter: Schmitt-Nilson Schraud Waibel Wohlfrom Patentanwälte Partnerschaft mbB

(56) Entgegenhaltungen:
- DE-A1- 2 947 363
- DE-A1-102016 001 140
- US-A- 4 454 893
- US-B2- 7 066 177

## Beschreibung

Die vorliegende Erfindung betrifft ein Expirationsventil aufweisend einen Ventil - körper, in dem ein Fluideinlass und ein Fluidauslass für Atemluft eines Patienten ausgebildet sind, wobei der Ventilkörper ferner eine Auflage aufweistmit einer Oberseite, an der eine Ventilmembran mit einer an ihrem äußeren Rand ausgebil - deten Anlagefläche auflegbar ist, wobei die Oberseite der Auflage uneben ausge - bildet ist.

Dieses Expirationsventil wird bei einem Beatmungsgerät zur maschinellen Beatmung von Patienten eingesetzt. Die vorliegende Erfindung betrifft auch ein sol - ches Beatmungsgerät.

Außerdem betrifft die Erfindung ein Verfahren zum Zusammenbau eines solchen Expirationsventils mit einem Beatmungsgerät, bei welchem eine Ventilmembran mit einer entlang ihres Außenumfangs ausgebildeten Anlagefläche in Anlage ge - bracht wird mit einer Oberseite einer an einem Ventikörper des Expirationsventils ausgebildeten Auflage und das Expirationsventil an dem Beatmungsgerät befes - tigt wird, wobei der Ventilkörper durch Spannkräfte verformt wird, wobei die Oberfläche der am Ventilkörper ausgebildeten Auflage in einer solchen Weise un - ebenausgebildet ist, dass die beim Befestigen des Expirationsventils am Beat - mungsgerät durch Spannkräfte erzeugte Verformung des Ventilkörpers dazu führt, dass die Oberseite der Auflage eben wird.

Bei der maschinellen Beatmung wird einem Patienten Atemluft auf maschinellem Wege, in der Regel unter einem Überdruck zugeführt. Die maschinelle Beatmung kann die eigene Atmung des Patienten dabei unterstützen oder vollständig er - setzten. Das Zuführen der Beatmungsluft erfolgt in aufeinander folgenden Beat - mungszyklen. Jeder Beatmungszyklus hat eine Inspirationsphase mit nachfolgender Expirationsphase. Dabei wird die Beatmungsluft während der Inspirationsphase eines jeweiligen Beatmungszyklus zugeführt, in der Regel maschinell oder je - denfalls maschinell unterstützt, und mit einem Überdruck gegenüber dem in den Atemwegen des Patienten herrschenden Druck. Das Ausatmen erfolgt während einer nachfolgenden Expirationsphase des Beatmungszyklus. Hierbei erfolgt in der Regel keine Beaufschlagung der Atemwege mit Überdruck oder Unterdruck. Vielmehr soll das Ausatmen passiv erfolgen durch Entspannen der Atemwege ge - genüber dem Umgebungsdruck, wobei eigene Ausatmungsanstrengungen des Patienten soweit als möglich unterstützt werden sollen. Das Ausatmen der Atemluft und die Abgabe der Atemluft an die Umgebung soll mit Hilfe des hier be - schriebenen Expirationsventils erfolgen. Das Expirationsventil ist im Beatmungssystem in einer Leitung für ausgeatmete Luft angeordnet, in der Regel am stromabwärts liegenden Ende dieser Leitung. Auf seiner vom Patienten abgewandten Seite herrscht in der Regel Umgebungsdruck. Wird es während einer Expirations - phase mit Überdruck gegenüber diesem Druck von der Seite des Patienten her beaufschlagt, so soll das Expirationsventil öffnen und eine Abgabe der ausgeat - meten Luft an die Umgebung ermöglichen. Das Expirationsventil soll dabei Be - ginn und Ende von Expirationsphasen zuverlässig zu erkennen und entspre - chend reagieren. Hierzu dient eine Ventilmembran, die je nachdem, ob auf ihrer den Atemwegen zugewandten Seite ein genügend großer Überdruck gegenüber ihrer der Umgebung zugewandten Seite herrscht, einen Fluiddurchgang zwischen den Atemwegen der Umgebung freigibt oder verschließt.

DE 10 2016 001140 A1 zeigt ein Ausatmungsventil oder ein Einatmungsventil für ein Beatmungsgerät, um eine Fluidstrom zu steuern, welches einen Fluideinlass und einen Fluidauslass aufweist. Zwischen dem Fluideinlass und dem Fluideinlass ist eine Membran und ein Ventilsitzkrater angeordnet, wobei die Membran relativ zu dem Ventilsitzkrater beweglich ist.

Dokumente DE 2947363, US 4,454,893, US 7,066,177, WO 90/08566 zeigen weitere bekannte Ausatemventile.

In der Vergangenheit trat häufig das Problem auf, dass das Expirationsventil im Betrieb unangenehm laute Geräusche verursachte. In manchen Fällen wurde sogar beobachtet, dass vom Expirationsventil oszillierende Druckschwankungen in der ausgeatmeten Luft verursacht oder verstärkt wurden, die letztendlich zu Fehl - steuerungen in der Beatmung führten. Bei diesem als Autotrigger oder Autocycle bekannten Phänomen werden vom Expirationsventil verursachte oder verstärkte Druckschwankungen vom Beatmungsgerät fälschlicherweise als Ende der Expira - tionsphase detektiert und das Beatmungsgerät leitet vorzeitig die nächste Inspirationsphase ein. Dies ist für den zu beatmenden Patienten sehr unangenehm und kann sogar zur Gefährdung des beatmeten Patienten führen. Das hier vorge - schlagene Expirationsventil soll solche Probleme beheben oder jedenfalls vermin - dern.

Gegenüber den bisher verwendeten Expirationsventilen erlaubt das eingangs um - schriebene erfindungsgemäße Expirationsventil eine verbesserte Fixierung der Ventilmembran entlang ihres Umfangs am Ventilkörper. Der Ventilkörper ist dabei so ausgestaltet, dass durch das Befestigen des Expirationsventils an einem Beat-mungsgerät bedingte Verwerfungen und Falten am Umfang der Ventilmembran besser unterdrückt werden können. Zu diesem Zweck ist die am Ventilkörper ausgebildete Auflage für die Ventilmembran in bestimmter Weise uneben gestaltet. Wegen dieser unebenen Gestaltung liegt die Ventilmembran beim Einbau in das Expirationsventil - wobei das Expirationsventil noch nicht am Beatmungsgerät angebracht ist - zunächst nicht flächig mit ihrer am Umfang gebildeten Anlageflä - che auf der Oberseite der Auflage auf, sondern es entstehen einzelne Berüh - rungsstellen und in Umfangsrichtung zwischen den Berührungsstellen ausgebil - dete Hohlräume zwischen Ventilmembran und Auflage. Erst wenn das Expirati - onsventil dann am Beatmungsgerät befestigt wird, entstehen von den Befesti - gungsstellen ausgehende Spannungen im Ventilkörper und eine gewisse Defor - mation des Ventilkörpers. Die Unebenheit der Oberfläche der Anlagefläche ist so gewählt, dass diese Deformation des Ventilkörpers die Unebenheit der Oberseite der Auflage kompensiert. Nach Befestigen des Expirationsventils am Beatmungsgerät liegt die Oberseite der Auflage in einer ebenen Fläche und es entsteht ein flächiger Kontakt zwischen der am Umfang der Ventilmembran gebildeten Anlage - fläche und der Auflage. Damit lässt sich durch die uneben gestaltete Oberfläche der Anlage erreichen, dass die Ventilmembran im eingebauten Zustand entlang ihres Umfangs satt und ohne Falten eingeklemmt ist. Diese Maßnahme hilft sicherzustellen, dass die Ventilmembran luftdicht an ihrer Auflage anliegt, was die Betriebssicherheit des Expirationsventils verbessert. Es hat sich gezeigt, dass dies insbesondere auch eine luftdichte Anlage der Ventilmembran an ihrem um den Fluideinlass herum ausgebildeten Ventilsitz verbessert. Damit reagiert ein erfindungsgemäßes Expirationsventil feinfühliger auf den Beginn und das Ende der Expirationsphase.Außerdem ist die Geräuschentwicklung im Betrieb des Expirationsventils geringer als bei herkömmlichen Expirationsventilen. Zudem gelingt es, das Phänomen des Autotrigger oder Autocycle besser zu beherrschen.

Das Expirationsventil ist so ausgebildet, dass Atemluft beim Ausatmen, also während der Expirationsphase, durch den nunmehr geöffneten Fluideinlass in das Ex - pirationsventil, insbesondere in ein vom Expirationsventil umschlossenes Ventilplenum, eindringen kann. Während der Inspirationsphase ist der Fluideinlass vorzugsweise verschlossen.

Der Fluidauslass ist so ausgebildet, dass ausgeatmete Atemluft in eine Umge - bung abgebbar ist.

Im zusammengebauten Zustand weist das Expirationsventil ferner eine dem Fluideinlass zugeordnete Ventilmembran auf, die mindestens zwischen einer den Fluideinlass verschließenden Stellung und einer den Fluideinlass öffnenden Stel - lung verlagerbar ist. Die Ventilmembran kann ohne eigenen Antrieb ausgebildet sein, so dass sie lediglich durch Druckunterschiede zwischen ihrer dem Fluideinlass zugewandten Seite und ihrer vom Fluideinlass abgewandten Seite zwischen Verschlussstellung und Freigabestellung verlagerbar ist. Zusätzlich oder alternativ kann aber auch ein Aktor vorgesehen sein, der die Ventilmembran zwischen Verschlussstellung und Freigabestellung hin und her verlagert. Dieser Aktor wird vorzugsweise vom Beatmungsgerät angesteuert. Die Ventilmembran liegt mit der an ihrem äußeren Rand ausgebildeten Anlagefläche auf der der Oberseite der Aufla - ge auf. Das Expirationsventil weist insbesondere ferner ein Spannelement auf, beispielsweise einen Spannring oder Klemmring, welcher mit dem Ventilkörper derart zusammenwirkt, dass das Spannelement in Spanneingriff oder Klemmein - griff mit der Ventilmembran gelangt. Durch diesen Spanneingriff oder Klemmein - griff, der insbesondere am äußeren Umfang der Ventilmembran durch Klemmen mit dem Spannelement erfolgt, wird die Ventilmembran am Ventilkörper fixiert. Die Ventilmembran kann dabei durch das Spannelement so am Ventilkörper fixiert sein, dass sie bei Überdruck auf ihrer dem Fluideinlass zugeordneten Seite gegenüber ihrer dem Ventilplenum zugeordneten Seite in eine den Fluideinlass ge - genüber dem Ventilplenum freigebende Stellung bringbar ist. Solange das nicht der Fall ist, insbesondere bei Druckgleichheit oder Unterdruck auf ihrer dem Fluideinlass zugeordneten Seite gegenüber ihrer dem Ventilplenum zugeordneten Sei - te verbleibt die Ventilmembran in einer den Fluideinlass gegenüber dem Ventilple - num verschließenden Stellung.

Uneben ausgebildet bedeutet, dass die Oberseite der Auflage in einer gedachten gewölbten Fläche liegt. Dies bedeutet umgekehrt, dass es keine gedachte Ebene gibt, die die Oberseite der Auflage vollständig enthält. Die gedachte Ebene bzw. die gedachte gewölbte Fläche überdeckt eine Oberseite des Ventilkörpers. Sie liegt insbesondere orthogonal zu einer Längsachse des Ventilkörpers oder im Wesentlichen parallel zu einem radial äußeren Rand des Ventilkörpers. Die Venti - lachse kann parallel zu einer Fluideinlassachse sein. Würde man eine ebene Flä - che auf die Oberseite der Auflage auflegen, so würde diese ebene Fläche nicht die gesamte Oberseite der Auflage berühren, sondern es würden nur einzelne Stellen der ebenen Fläche Kontakt mit der Oberseite der Auflage haben.

Diese Angaben beziehen sich auf das Expirationsventil in einem Zustand vor Ein - bau in ein Beatmungsgerät, wenn der Ventilkörper nicht vorgespannt ist.

Jedenfalls im nicht in ein Beatmungsgerät eingebauten Zustand des Expirations - ventils liegt also die Ventilmembran - diese hat eine ebene Gestalt - nicht ent - lang ihres gesamten Umfangs an der Oberseite der Auflage auf. Es gibt vielmehr einzelne Berührungsstellen zwischen Ventilmembran und Auflage entlang des Umfangs der Ventilmembran. Die Unebenheit der Oberseite der Auflage ist nun so ausgebildet, dass dann, wenn das Expirationsventil in das Beatmungsgerät eingebaut ist und der Ventilkörper unter Spannung steht, so dass er sich defor - miert, die Oberseite der Auflage in einer ebenen Fläche zu liegen kommt und die Ventilmembran nun mit ihrer Anlagefläche flächig auf der Oberseite der Auflage aufliegt.

Der Ventilkörper kann insbesondere wenigstens eine Montagelasche aufweisen, die zum Eingriff mit einem Expirationsventilanschluss eines Beatmungsgeräts ausgebildet ist.

Die Montagelasche soll insbesondere ein Eingriffselement eines bajonettartigen Verschlusses des Expirationsventils am Beatmungsgerät bilden. Das Beatmungs - gerät weist dann ein Gegeneingriffselement auf, mit dem die Montagelasche beim Einbau des Expirationsventils in Eingriff gelangt, um das Expirationsventil am Be - atmungsgerät zu halten. Insbesondere soll so das Expirationsventil mittels eines Bajonettverschlusses am Beatmungsgerät verriegelt sein, so dass beim Befestigen des Expirationsventils am Beatmungsgerät Spannkräfte entstehen, die den Ventilkörper etwas deformieren.

Der Ventilkörper kann insbesondere wenigstens zwei Montagelaschen aufweisen, die einander zugeordnet sind. Die Montagelaschen können insbesondere am ra - dial äußeren Rand des Ventilkörpers um einen Umfang des Ventilkörpers herum angeordnet sein. Sie stehen insbesondere jeweils in radialer Richtung vom jewei - ligen radial äußeren Rand des Ventilkörpers hervor, um jeweilige Eingriffsflächen mit einem entsprechenden Gegeneingriffselement am Beatmungsgerät zu bilden. Bringt man die Montagelaschen in Eingriff am Beatmungsgerät, wird der Ventil - körper auf den jeweils zugeordneten Seiten, an denen sich die Montagelaschen befinden, zum Beatmungsgerät hin geführt. Im Zuge dieser Zwangsbewegung entsteht eine Spannung im Ventilkörper und der obere Rand des Ventilkörpers biegt sich auf den Seiten, an denen die Montagelaschen angeordnet sind, zum Beatmungsgerät hin. Dieser Effekt liegt im Millimeterbereich, er kann sogar nur Bruchteile eines Millimeters betragen. Dennoch hat sich gezeigt, das hierbei der Sitz der Ventilmembran beeinträchtigt werden kann und insbesondere Undichtig - keiten am Umfang der Ventilmembran auftreten können. Die unebene Ausgestal - tung der am Ventilkörper vorgesehenen Auflage für die Ventilmembran soll die - sem Effekt Rechnung tragen. Nach Ineingriffbringen der Montagelaschen mit dem Beatmungsgerät soll die Oberseite der Auflage flach sein, also in einer Ebene lie - gen.

Insbesondere kann der Ventilkörper ein Paar von Montagelaschen aufweisen, die einander gegenüber liegen. Die beiden Montagelaschen können dann insbeson - dere in einem Winkel von 180° zueinander auf einander gegenüberliegenden Sei - ten am radial äußeren Rand des Ventilkörpers ausgebildet sein. Wenn mehr als zwei Montagelaschen vorhanden sind, gilt das oben Gesagte entsprechend, mit der Maßgabe, dass dann die beim Befestigen des Expirationsventils am Beatmungsgerät auf den Ventilkörper ausgeübte Spannung sich entsprechend der An - ordnung der Montagelaschen verteilt und dementsprechend auch die Oberseite der Auflage entsprechend der Lage der Montagelaschen uneben auszugestalten ist. Im Folgenden wird zur besseren Veranschaulichung der Beschreibung von zwei einander gegenüber liegenden Montagelaschen ausgegangen, wobei des sich versteht, dass im Fall von mehr also zwei Montagelaschen entsprechende Anpassungen vorzunehmen sind.

Die Oberseite der Auflage kann in einer sich bezogen auf ein vom Ventilkörper umgebenes Ventilplenum nach außen gewölbten gedachten Fläche liegen. Die gedachte Fläche überdeckt eine Oberseite des Ventilkörpers, so dass sie - wäre sie eine reale Fläche - eine Decke für den Ventilkörper bilden würde. Die gedach - te Fläche begrenzt also das vom Ventilkörper umschlossene Ventilplenum nach oben hin, d.h. zu einer einem Ventilboden gegenüber liegenden Seite hin. Insbe - sondere liegt die nach außen gewölbte gedachte Fläche bezogen das Ventilple - num weiter außen als eine gedachte Ebene durch wenigstens zwei verschiedene Punkte auf der Oberseite der Auflage an Seiten, an denen Montagelaschen vor - handen sind.

Insbesondere kann die Oberseite der Auflage in einer konvex gewölbten gedach - ten Fläche liegen. Bei einer konvex gewölbten gedachten Fläche verläuft eine zwei in der gedachten Fläche liegende Punkte verbindende Linie durch das Ven - tilplenum. Es mag dabei durchaus vorkommen, dass eine solche Linie auch in der gedachten Fläche verläuft, wenn die gedachte Fläche aus mehreren ebenen Teil - stücken zusammengesetzt ist oder in einer Richtung linear verläuft (z.B. im Falle einer zylindrischen Fläche parallel zur Achsrichtung des Zylinders). Dann verlau - fen jeweils Linien, die die einzelnen ebenen Teilstücke verbinden, oder Linien im Winkel zu der linearen Richtung durch das von der konvex gewölbten gedachten Fläche umschlossene Ventilplenum.

Insbesondere kann die Oberseite der Auflage sich ausgehend von der wenigs - tens einen Montagelasche nach außen auswölben. An Stellen entlang des Um - fangs der Auflage, an denen Montagelaschen vorgesehen sind, soll die Auswöl - bung der Oberseite der Auflage am kleinsten sein, weil dort die Deformierung des Ventilkörpers beim Befestigen des Expirationsventils am Beatmungsgerät am stärksten ist.

Dementsprechend kann die Auswölbung der Auflage in einem zwischen den Mon - tagelaschen liegenden Bereich am größten sein. An Stellen entlang des Umfangs der Auflage, die am weitesten von Montagelaschen entfernt sind, ist die Deformierung des Ventilkörpers beim Befestigen des Expirationsventils am Beatmungsgerät am geringsten. Damit soll an diesen Stellen eine möglichst geringe Kompensation der Lage der Oberseite der Auflage erfolgen.

Beispielsweise kann die Auswölbung der Auflage entlang einer zwischen den bei - den Montagelaschen verlaufenden Linie am größten sein. Eine solche Linie schneidet eine Verbindungslinie zwischen den beiden Montagelaschen über das Ventilplenum hinweg orthogonal.

Insbesondere, dann wenn zwei Montagelaschen vorhanden sind, die sich an zwei einander gegenüber liegenden Seiten des Ventilkörpers in einem Winkelabstand von 180° zueinander befinden, kann die gewölbte gedachte Fläche beispielswei - se eine Mantelfläche eines Zylinders sein. Die Achse des Zylinders kann dann orthogonal zu einer Verbindungslinie zwischen den beiden zugeordneten Montage - laschen sein.

Wegen der beim Befestigen des Expirationsventils an Stellen, an den Montagela - schen sind, auf den den Ventilkörper ausgeübten Spannung biegt sich auch die obere Kante des Ventilkörpers in derselben Weise an diesen Stellen zu einer Ge - genseite am Beatmungsgerät hin. Zur Kompensation dieses Effektes ist es güns - tig, auch den Ventilkörper in entsprechender Weise auszubilden wie die Auflage für die Ventilmembran. Die obere Kante des Ventilkörpers soll nach Befestigen des Expirationsventils am Beatmungsgerät an einer Gegenfläche des Beat - mungsgeräts anliegen und zwar in einer möglichst flächigen Weise, um eine mög - lichst dichte Anlage der Ventilmembran (beispielsweise durch Ausüben von Druck auf eieine Oberseite der Ventilmembran, ggf. über einen Spannring) an ihrer Auf - lage zu gewährleisten. Deshalb kann vorgesehen sein, dass der Ventilkörper eine obere Kante aufweist, welche in einer sich bezogen auf ein vom Ventilkörper um - gebenes Ventilplenum nach außen gewölbten gedachten Fläche liegt.

Die vorangehend für die Oberseite der Auflage angestellten Überlegungen gelten für die Ausgestaltung der oberen Kante des Ventilkörpers in entsprechender Wei - se. Sie sollen deshalb nicht nochmals wiederholt werden, sondern stattdessen soll auf die obigen Ausführungen verwiesen werden mit der Maßgabe, dass an - stelle der Oberseite der Auflage nunmehr von der Oberen Kante des Ventilkörpers die Rede ist. Insbesondere kann die obere Kante des Ventilkörpers in einer sich bezogen auf ein vom Ventilkörper umgebenes Ventilplenum konvex gewölb - ten gedachten Fläche liegen. Dabein kann die obere Kante des Ventilkörpers sich ausgehend von der wenigstens einen Montagelasche auswölben. Die Auswöl - bung der oberen Kante des Ventilkörpers kann in einem zwischen den beiden Montagelaschen liegenden Bereich am größten sein. Die Auswölbung der oberen Kante des Ventilkörpers kann insbesondere entlang einer zwischen den beiden Montagelaschen (50) verlaufenden Linie am größten sein. Auch hier kann die ge - wölbte gedachte Fläche eine Mantelfläche eines Zylinders sein.

Es ist in aller Regel sinnvoll, die unebene Form der Oberseite der Auflage und die unebene Form der oberen Kante des Ventilkörpers aufeinander abzustimmen. Schließlich soll für beide erreicht werden, dass nach Befestigen des Expirations - ventils am Beatmungsgerät die Oberseite bzw. obere Kante in einer ebenen Flä - che liegt. Insbesondere kann vorgesehen sein, dass die Oberseite der Anlage und der obere Rand des Ventilkörpers sich zueinander ausgerichtet nach außen wölben. Hierbei können insbesondere die Orte minimaler Auswölbung und maximaler Auswölbung aufeinander abgestimmt sein. Dies Stellen können insbeson - dere in Bezug auf eine das Ventilplenum überdeckende Ebene an derselben Stel - le liegen.

Es mag dabei manchmal sinnvoll sein, wenn sich die Oberkante der Auflagebe in etwas stärkerem Maße oder in etwas schwächerem Maße auswölbt als die obere Kante des Ventilkörpers. In vielen Fällen wird man jedoch finden, dass die beabsichtigte Kompensation der Deformierung des Ventilkörpers unter der beim Eingriff der Montagelaschen mit den Beatmungsgerät erzeugten Spannung am bes - ten gelingt, wenn die Oberseite der Anlage und die obere Kante des Ventilkörpers sich in etwa gleichermaßen auswölben.

Insbesondere können die Oberseite der Anlage und die obere Kante des Ventil - körpers in zueinander parallelen gedachten Flächen liegen.

In besonderer Ausgestaltung kann die Montagelasche eine Eingriffsfläche zum Eingriff mit einem am Beatmungsgerät vorgesehenen Gegeneingriffselement auf - weist, wobei die Eingriffsfläche mit einer Einlaufschräge versehen ist, die zu der Auswölbung der Oberseite der Anlage abgestimmt ist.

Wie bei einem Bajonetteingriff üblich, wird die Eingriffsfläche in der Regel im We - sentlichen parallel zu einer oberen Kante der Montagelasche ausgebildet sein. In ihrem vorderen Bereich kann man eine Einlaufschräge vorsehen. Im Bereich der Einlaufschräge verläuft die Eingriffsfläche dann in einem Winkel, insbesondere ei - nem spitzen Winkel, zu der oberen Kante der Montagelasche. Der Winkel ist so gewählt, dass eine Dicke zwischen Eingriffsfläche und oberen Kante der Monta - gelasche mit zunehmendem Abstand vom Beginn der Eingriffsfläche zunimmt. Damit lässt sich eine gewisse Spannung beim Ineingriffbringen der Montagelasche mit dem Gegeneingriffselement am Beatmungsgerät erzeugen, was zu einem gewissen Kraftschluss und damit einem festen und sicheren Sitz des Expira - tionsventils am Beatmungsgerät verhilft.

Die Einlaufschräge ist so ausgebildet, dass beim Einspannen der Montagelasche am Beatmungsgerät eine Vorspannung erzeugt wird, die auf die obere Kante des Ventilkörpers auf der Seite der Montagelasche einwirkt. Damit einher geht eine gewisse Deformation des Ventilkörpers auf der der Montagelasche zugewandten Seite gegenüber Seiten oder Stellen weiter abseits der Montagelasche. Insbesondere wird die obere Kante des Ventilkörpers durch Bewegung des Gegeneingriffs - elements entlang der Einlaufschräge näher zum Beatmungsgerät hin geführt. An - dererseits liegen weiter von der Montagelasche entfernte Stellen bereits an einer Kontaktfläche des Beatmungsgeräts an und können sich nicht weiter annähern. Im Ergebnis entsteht eine Verbiegung der oberen Kante des Ventilkörpers, wobei die Verbiegung zu der Montagelasche hin immer stärker wird. Dadurch entsteht eine Vorspannung des Ventilkörpers, die mit zunehmender Bewegung entlang der Einlaufschräge zunimmt. Die Einlaufschräge ist so gewählt, dass beim Einspan - nen eine Vorspannung erzeugt wird, die gerade groß genug ist, um die obere Kante des Ventilkörpers im eingespannten Zustand eben zu machen, d.h. dass die obere Kante des Ventilkörpers im eingespannten Zustand in einer das Ventil - plenum überdeckenden Ebene liegt. Dann liegt die obere Kante des Ventilkörpers flächig an einer Gegenfläche des Beatmungsgeräts an. Gleichermaßen verbiegt sich auch die Auflage für die Ventilmembran. Damit die Oberseite dieser Auflage im Beatmungsgerät befestigten Zustand des Expirationsventils ebenfalls eben ist, ist es günstig, wenn die Oberseite der Auflage in demselben Maße uneben ist wie die obere Kante des Ventilkörpers. Die Einlaufschräge der Montagelasche ist demgemäß auf die Auswölbung der Auflage abgestimmt.

Um die Vorspannung für den Ventilkörper möglichst genau einstellen zu können, ist es günstig, wenn das Ende der Einlaufschräge möglichst gut definiert ist. Hierzu kann vorgesehen sein, dass die Eingriffsfläche einen auf die Einlaufschräge folgenden nicht abgeschrägten Bereich aufweist.

Im nicht abgeschrägten Bereich ist die Eingriffsfläche parallel zu der oberen Kan - te der Montagelasche. Eine einmal erreichte Vorspannung und Verbiegung des Ventilkörpers änderst sich nicht mehr. Damit kann die Vorspannung und Verbiegung gut reproduzierbar eingestellt werden, weil es nicht genau darauf ankommt, bis zu welcher Stelle des nicht abgeschrägten Bereichs die Montagelasche ge - langt. Zudem kann noch die Eingriffsfläche an ihrem Ende mit einem Anschlag versehen sein.

Der Ventilkörper kann insbesondere aus Kunststoffmaterial hergestellt sein. Es bietet sich beispielsweise an, den Ventilkörper im Spritzgussverfahren herzustellen. Dann ist der Ventilkörper ein Spritzgussteil. Beispielsweise kann der Ventilkörper einschließlich des Ventilbodens aus einem spritzgussfähigen Kunststoffmaterial hergestellt sein, etwa Polyethylen, Polypropylen oder einem anderen thermoplastischen Kunststoffmaterial.

Es ist günstig, wenn die Auflage integral mit dem Ventilkörper ausgebildet ist. Integral ist so verstehen, dass die Auflage und der Ventilkörper als ein Bauteil hergestellt sind, beispielsweise dass die Auflage im selben Spritzgussprozess herge - stellt sind wie der Ventilkörper, insbesondere als Einkomponentenspritzgussteil.

In Ausführungsformen bildet der Ventilkörper ein topfförmiges Ventilplenum, wel - ches wenigstens zum Teil an seiner Unterseite von dem Ventilkörper, insbesondere von einem vom Ventilkörper gebildeten Ventilboden und ggf. von einem vom Ventilboden ausgehenden und ebenfalls vom Ventilkörper gebildeten äußeren Rand, umschlossen ist und in das der Fluideinlass mündet. An seiner Oberseite kann das Ventilplenum von einer einen Deckel bildenden Ventilmembran begrenzt sein. Das Ventilplenum bildet einen Hohlraum, der über den Fluid einlass mit Fluid (ausgeatmete Atemluft) beaufschlagbar ist und aus dem über den Fluidauslass das Fluid (ausgeatmete Atemluft) ausströmen kann. Insbesondere ist in dem Ventilplenum der Fluidströmungsweg zwischen dem Fluideinlass und dem Fluidauslass gebildet. Insbesondere ist das Ventilplenum wenigstens zum Teil an seiner Unterseite von dem Ventilboden begrenzt und der Fluideinlass mündet in den Ventilboden. Der Ventilboden kann sich dabei insbesondere tellerartig zwischen dem Fluideinlass und einem äußeren Rand des Ventilkörpers (insbesondere des Ventilplenums) erstrecken, wobei der äußere Rand des Ventilbodens zugleich den äußeren Rand des Ventilkörpers bildet. Mit dem Begriff "tellerartig" soll umschrieben ein, dass der Ventilboden sich, abgesehen von einer möglichen noch näher zu erläuternden rinnenartigen Vertiefung um den Fluideinlass herum, im Wesentlichen flach oder flach konisch zwischen dem Fluideinlass und einem äu - ßeren Rand erstreckt. Der Fluideinlass ist dann insbesondere in einem zentralen Teil des Ventilbodens ausgebildet. Der Fluideinlass kann insbesondere einen kreisförmigen Querschnitt aufweisen mit ebenfalls kreisförmiger Fluideintrittsfläche.

Insbesondere kann der Ventilboden von einem eine Fluideintrittsfläche definieren - den Rand des Fluideinlasses ausgehen. Es entsteht kein Absatz zwischen der Fluideintrittsfäche und dem Ventilboden. Auf diese Weise werden scharfkantige Begrenzungen des Fluideinlasses gegenüber dem Ventilplenum vermieden. Das fördert die Ausbildung einer möglichst ruhigen Strömung des Fluids in das Expirationsventil sowie durch das Expirationsventil während der Expirationsphase. Bei bekannten Expirationsventilen ragt demgegenüber der Fluideinlass in das Ventil - plenum hinein, es steht insbesondere der Fluideinlass ein Stück weit über den Ventilboden vor. Der Ventilboden geht dann nicht von dem die Fluideintrittsfläche definierenden Rand des Fluideinlasses aus und die Fluidströmung passiert einen Absatz beim Einströmen in das Ventilplenum, was die Ausbildung von Turbulenz und Strömungsabrissen in der in das Expirationsventil einströmenden Fluidströmung fördert.

Zur Reduzierung von Geräuschbildung und Druck-Rückwirkungen kann auch be - tragen, wenn das Ventilplenum entlang des Fluidströmungswegs zwischen Fluid - einlass und Fluidauslass möglichst strömungsgünstig ausgelegt ist. Die strömungsgünstige Form des Ventilplenums, insbesondere des Fluidströmungswegs kann insbesondere verbessert werden, wenn der Ventilboden ausgehend von ei - ner Innenseite des Fluideinlasses am Rand der Fluideintrittsfläche auf seiner dem Ventilplenum zugewandten Seite eine abgerundete Kontur aufweist. Die vom Flui - deinlass her in das Ventilplenum einströmende Fluidströmung gelangt so weitgehend unverwirbelt und ohne Strömumgsabrisse in den Fluidströmungsweg und strömt dem Fluidauslass zu. Dies vermindert Betriebsgeräusche bei geöffnetem Fluidauslass unmittelbar. Außerdem werden Druck-Rückschlage in die an dem Fluideinlass angeschlossene Atemluftleitung effektiv unterdrückt, so dass die Ge - fahr von Fehl-Erfassungen eines Endes der Expirationsphase durch das Beat - mungsgerät geringer wird.

In bestimmten Ausführungsformen kann der Fluidströmungsweg einen sich zum Fluidauslass hin erweiternden Querschnitt aufweisen. In dieser Ausgestaltung ist der Fluidströmungsweg besonders gut an den zum Fluidauslass hin immer größer werdenden Volumenstrom des während der Expiration durch das in dieser Phase geöffnete Expirationsventil strömenden Fluids (der ausgeatmeten Luft) ange - passt. Je größer der Volumenstrom, desto größer der im Fluidströmungsweg bereitstehende Querschnitt. Insbesondere verändern sich auf jeweils gleich große Winkelbereiche bezogene Teilvolumina des Fluidströmungswegs entlang eines Umfangs des Fluideinlasses, und zwar derart, dass diese Teilvolumina in Richtung zum Fluidauslass hin immer größer werden. Das vermindert die Ausbildung von Turbulenz und Störungen in der Fluidströmung und schafft die Möglichkeit, dass das Expirationsventil sehr viel zuverlässiger, aber gleichzeitig auch empfind - licher, auf Veränderungen der Strömung ausgeatmeter Luft in dem Atemwegen regieren kann. Druck-Rückschläge, die bei herkömmlichen Expirationsventilen häufig zu den genannten Autotrigger-Fehlsteuerungen führten, treten sehr viel weniger in Erscheinung.

Der Querschnitt des Fluidströmungswegs soll als Fläche des für die Fluidströmungzur Verfügung stehenden Raums in quer, insbesondere orthogonal, zur hauptsächlichen Strömungsrichtung der Fluidströmung zwischen Fluideinlass und Fluidauslass bei geöffnetem Expirationsventil stehenden Schnittebenen verstan - den werden.

In bestimmten Ausführungsformen kann vorgesehen sein, dass der Ventilboden sich in einem Bereich um den Fluideinlass herum in axialer Richtung auswölbt. Mit der Formulierung "in axialer Richtung auswölbt" soll dabei ausgedrückt werden, dass der Ventilboden in wenigstens einem eine Fluideinlassachse enthalten - den Schnitt einen konvexen Verlauf hat, und zwar in einem zwischen dem Rand des Fluideinlasses und dem Außenrand des Ventilbodens liegenden Bereich. Konvex bedeutet, dass in einem solchen Schnitt eine zwei Punkte auf dem Ventil - boden verbindende Linie durch das Ventilplenum verläuft. Der Ventilboden bildet demgemäß eine um den Fluideinlass umlaufende Rinne aus, deren Boden sich zwischen dem Rand des Fluideinlasses und dem Außenrand des Ventilbodens in axialer Richtung auswölbt. Der Querschnitt des Fluidströmungswegs zwischen dem Fluideinlass und dem Fluidauslass ergibt sich bei solcher Ausgestaltung jeweils als Fläche des Ventilplenums von der Fluideinlassachse ausgehenden und bis zum äußeren Rand des Ventilplenums reichenden radialen Schnitt. Im Gegensatz dazu ist der Ventilboden bei derzeit Verwendung findenden Expirationsventi - len flach konisch ausgebildet, er bildet also keine Auswölbung.

Insbesondere kann vorgesehen sein, dass die axiale Auswölbung eine sich um den Umfang des Fluideinlasses herum verändernde Tiefe hat. Die Tiefe der Aus - wölbung lässt sich dabei als ein in axialer Richtung gemessenen Abstand zwi - schen dem Ventilboden am Rand des Fluideinlasses und dem Ventilboden an der tiefsten Stelle der Auswölbung entlang eines Schnitts vom Rand des Fluideinlas - ses zum äußeren Rand des Ventilbodens bestimmen. Insbesondere soll die Tiefe der Auswölbung in Umfangsrichtung zu dem Fluidauslass hin zunehmen. Damit verändern sich auf jeweils gleich große Winkelbereiche bezogene Teilvolumina der Auswölbung entlang des Umfangs des Fluideinlasses, und zwar derart, dass diese Teilvolumina in Richtung zum Fluidauslass hin immer größer werden. In bestimmten Ausführungsformen kann die Tiefe der Auswölbung in Umfangsrichtung auf einer dem Fluidauslass gegenüber liegenden Seite am kleinsten sein und in Umfangsrichtung auf der zum Fluidauslass hin gelegenen Seite am größten sein. In bestimmten Ausführungsformen kann der Ventilkörper wenigstens eine um den Fluideinlass herum ausgebildete Versteifungsrippe aufweisen. Der Ventilkörper kann dabei insbesondere mehrere um den Fluideinlass herum ausgebildete Versteifungsrippen aufweisen. Die mehreren Versteifungsrippen können insbesonde - re in bestimmten Abständen zueinander um einen Umfang des Fluideinlasses herum aufeinander folgen. Ein einzige Versteifungsrippe kann beispielsweise als ein den Fluideinlass umgebender Vollkörper ausgebildet sein. Die Versteifungs - rippe(n) sind insbesondere auf einer Unterseite des Ventilbodens ausgebildet, also auf einer vom Fluidströmungsweg abgewandten Seite des Ventilbodens, so dass sie die Fluidströmung zwischen dem Fluideinlass und dem Fluidauslass nicht stören.

Die Versteifungsrippe bzw. die Versteifungsrippen sind geeignet, den Ventilboden zu versteifen, insbesondere in dem sich auswölbenden Bereich, der den Fluidein - lass umgibt. Vorsehen von Versteifungsrippe(n) führt zu einer Erhöhung des Flä - chenträgheitsmoments des Ventilkörpers, insbesondere des Ventilbodens, über den Querschnitt des Fluidströmungswegs hinweg. Der Ventilkörper wird in diesem Bereich verwindungsstreifer, so dass die in das Ventilplenum einströmende und entlang des Fluidströmungswegs strömende Fluidströmung weniger Schwingungen des Ventilkörpers, insbesondere des Ventilbodens, anregt. Dadurch wird vor allem Geräuschentwicklung unterdrückt, es entstehen aber auch viel weniger Druck-Rückwirkungen, die sich in der am Fluideinlass angeschlossenen Atemwegsleitung bemerkbar machen.

Es ist günstig, wenn die Versteifungsrippen integral mit dem Ventilkörper ausgebildet sind. Integral ist so verstehen, dass die Versteifungsrippen und der Ventil - körper als ein Bauteil hergestellt sind, beispielsweise dass die Versteifungsrippen im selben Spritzgussprozess hergestellt sind wie der Ventilkörper, insbesondere als Einkomponentenspritzgussteil.

Günstigerweise können die Versteifungsrippen bezogen auf einen Rand des Flui - deinlasses nach außen weg verlaufen. Nach außen weg soll dabei die Richtung der Versteifungsrippen bezeichnen, die so angeordnet sind, dass sie den Bereich des Ventilbodens zwischen dem Rand des Fluideinlasses und dem äußeren Rand des Ventilbodens möglichst effektiv versteifen. Insbesondere können die Verstei - fungsrippen bezüglich einer Mitte eines Querschnitts des Fluideinlasses in radialer Richtung verlaufen. Es ist aber auch denkbar, dass die Versteifungsrippen nicht genau in radialer Richtung verlaufen, sondern zusätzlich in Umfangsrich - tung, beispielsweise spiralförmig auf den äußeren Rand des Ventilbodens zu. Ins - besondere könne die Versteifungsrippen außerhalb des Rands des Fluideinlas - ses angeordnet sein.

Die Versteifungsrippen sind geeignet, den Ventilboden in dem sich auswölbenden Bereich zu versteifen. Dadurch lässt sich Geräuschentwicklung bei strömender Atemluft verringern, weil Schwingungen des Ventilbodens unterdrückt werden.

Die Versteifungsrippen können erste Versteifungsrippen und zweite Versteifungs - rippen umfassen. Die ersten Versteifungsrippen sind innere Versteifungsrippen, die radial innerhalb der zweiten Versteifungsrippen angeordnet sind. Die zweiten Versteifungsrippen sind demgemäß äußere Versteifungsrippen. Die ersten Versteifungsrippen verlaufen insbesondere zwischen dem Rand des Fluideinlasses und dem sich auswölbenden Bereich des Ventilbodens. Die zweiten Versteifungs - rippen verlaufen insbesondere zwischen dem sich auswölbenden Bereich des Ventilbodens und einem äußeren Rand des Ventilbodens. Insbesondere enden die zweiten Versteifungsrippen in einem am äußeren Rand des Ventilbodens aus - gebildeten flanschartigen Ansatz. Der Ansatz kann beispielsweise mindestens einen um den Rand des Ventilbodens umlaufenden Absatz aufweisen, vorzugs - weise mindestens zwei im Abstand zueinander angeordnete Absätze, wobei die zweiten Versteifungsrippen in den Absatz, bzw. einen inneren der Absätze, übergehen.

Wie bereits angesprochen, kann der Fluideinlass insbesondere in einem zentra - len Bereich des Ventilplenums angeordnet sein. Der Fluidauslass kann bezogen auf den Fluideinlass weiter außen angeordnet sein, insbesondere in radialer Rich - tung weiter außen, insbesondere an einem äußeren Rand des Ventilplenums. Der Querschnitt des Fluidströmungswegs zwischen dem Fluideinlass und dem Fluidauslass ergibt sich bei zentraler Anordnung des Fluideinlasses jeweils als Querschnittsfläche des Ventilplenums in einem jeweiligen von der Fluideinlassachse ausgehenden und bis zum äußeren Rand des Ventilplenums reichenden radialen Schnitt.

In bestimmten Ausführungsformen kann der Ventilkörper einen Fluideinlassteil aufweisen, in dem der Fluideinlass ausgebildet ist. Insbesondere kann der Fluideinlassteil integral mit dem Ventilkörper ausgebildet sein, also ebenfalls als Spritzgussteil für den Fall, dass der Ventilkörper ein Spritzgussteil ist. Der Fluideinlassteil kann sich insbesondere von dem Ventilboden weg erstrecken, und zwar insbesondere von einer Unterseite des Ventilbodens weg. Der Fluideinlass - teil kann sich beispielsweise orthogonal zu dem Ventilboden erstrecken. Die Flui - deinlassachse bezeichnet eine gedachte Linie in Längsrichtung des Fluidein - lasssteils, die bezüglich des Fluideinlassteils zentral liegt. Bei orthogonal zum Fluideinlass stehender Fluideintrittsfläche steht die Fluideinlassachse orthogonal zu der durch den Fluideinlass definierten Fluideintrittsfläche und liegt bezüglich der Fluideintrittsfläche zentral. In bestimmten Ausführungsformen kann der Flui - deinlasssteil einen ersten zapfenartigen Ansatz ausbilden, der zum Anschluss ei - nes Atemluftschlauches ausgebildet sein kann. Beispielsweise kann der erste zapfenartige Ansatz zylindrisch oder konisch geformt sein und insbesondere als ein zylindrischer oder konischer Rohrstutzen ausgebildet sein, in welchem der Fluideinlass ausgebildet ist. Der Fluideinlass kann insbesondere einen kreisförmi - gen Querschnitt aufweisen.

Weiterhin kann insbesondere der Ventilkörper einen Fluidauslassteil bilden, in dem der Fluidauslass ausgebildet ist. Der Fluidauslassteil kann insbesondere einen zweiten zapfenartigen Ansatz ausbilden. Der Fluidauslassteil kann insbe - sondere integral mit dem Ventilkörper ausgebildet sein, beispielsweise ebenfalls als Spritzgussteil. Der zweite zapfenartige Ansatz kann insbesondere zylindrisch oder konisch geformt sein. Falls gewünscht, kann der zweite zapfenartige Ansatz beispielsweise als ein zylindrischer oder konischer Rohrstutzen ausgebildet sein, in welchem der Fluidauslass ausgebildet ist. Oft wird der zweite Ansatz aber unmittelbar in die Umgebung münden. Der Fluidauslass kann insbesondere einen kreisförmigen Querschnitt aufweisen.

Der sich auswölbende Bereich des Ventilbodens, welcher den Fluidströmungsweg zwischen Fluideinlass und Fluidauslass definiert, kann in den Fluidauslassteil übergehen. Der sich auswölbende Bereich des Ventilbodens kann insbesondere in eine Seitenwand des Fluidauslassteils übergehen. Insbesondere hat am Übergang zum Fluidauslassteil die durch den sich auswölbenden Ventilboden definier - te Rinne eine maximale Tiefe.

Wie der Fluideinlassteil kann sich der Fluidauslassteil insbesondere vom Ventilbo - den weg erstrecken, insbesondere von einer Unterseite des Ventilbodens weg. Der Fluideinlassteil kann sich orthogonal zu dem Ventilboden erstrecken.

Die Erfindung wird nachfolgend anhand eines Ausführungsbeispiels unter Bezug - nahme auf die Figuren näher erläutert. Es zeigt:
Fig. 1: Eine Beatmungsmaschine zur maschinellen Beatmung von Patienten, mit Steuereinheit, Monitor, sowie Schläuchen für zugeführte Beatmungsluft und für ausgeatmete Luft;
Fig. 2: Ein Expirationsventil gemäß einer Ausführungsform in Schnittansicht;
Fig. 3: Den Ventilkörper des Expirationsventils aus Fig. 2 in einer perspektivischen Ansicht von unten; und
Fig. 4: Den Ventilkörper des Expirationsventils aus Fig. 2 in einer perspektivischen Ansicht von oben.

In allen Figuren werden für jeweils gleiche oder funktionsgleiche Bauteile diesel - ben Bezugszeichen verwendet. Eine detaillierte Beschreibung solcher Bauteile erfolgt jeweils nur für die erste Figur, in der das jeweilige Bezugszeichen erscheint. Es versteht sich, dass dieselbe Beschreibung auch für alle anderen Figu - ren gilt, in denen das mit demselben Bezugszeichen versehene Bauteil zu sehen ist, es sei dann, dass ausdrücklich anderes vermerkt ist.

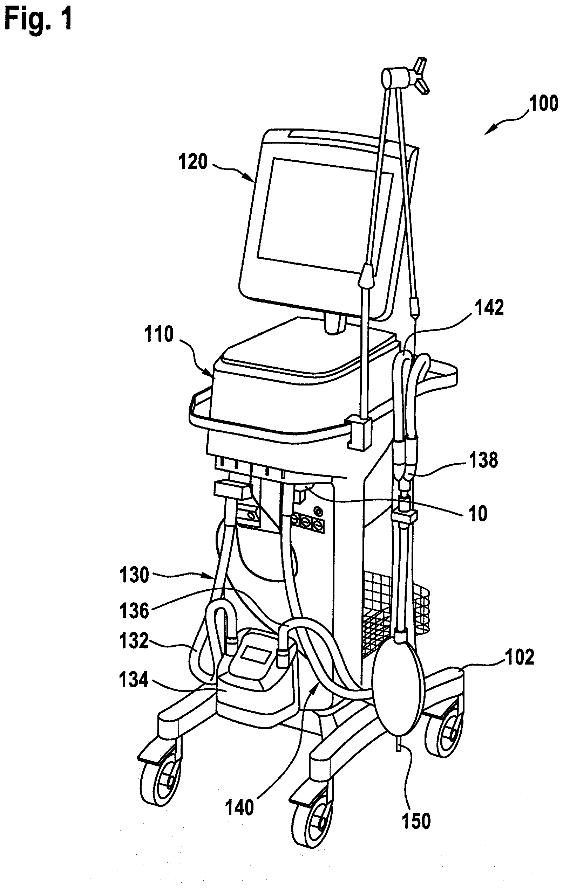

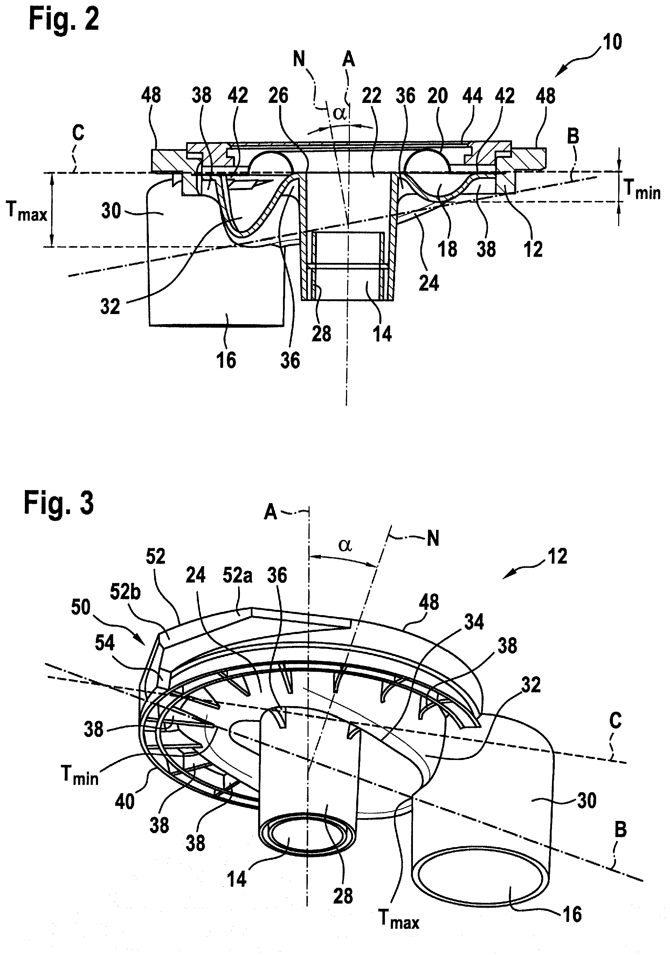

Fig. 1 zeigt eine Beatmungsmaschine 100 zur maschinellen Beatmung von Patienten. Die Beatmungsmaschine 100 umfasst ein auf Rollen fahrbares Gestell 102 mit Steuereinheit 110 und Monitor 120 sowie eine Leitung 130 für von der Maschi - ne zugeführte Beatmungsluft und eine Leitung 140 für ausgeatmete Luft. Die Lei - tung 130 für zugeführte Beatmungsluft umfasst einen von der Steuereinheit 110 ausgehenden ersten Beatmungsluftschlauch 132, der zu einer Befeuchtungsein - heit 134 führt, wo die Beatmungsluft durch ein Wasserreservoir geleitet wird. Von der Befeuchtungseinheit 134 führt ein zweiter Beatmungsluftschlauch 136 zu ei - nem T-Stück 138. Von dem T-Stück gelangt die von der Beatmungsmaschine 100 während einer Inspirationsphase des Beatmungszyklus bereitgestellte Beatmungsluft über eine Leitung 150 zum Patienten. Über die Leitung 150 gelangt auch die vom Patienten während einer Expirationsphase des Beatmungszyklus ausgeatmete Luft zurück zur Beatmungsmaschine 100. Hierzu zweigt von dem T-Stück 138 ein weiterer Atemluftschlauch 142 ab, der zu der Leitung 140 für aus - geatmete Luft gehört. Der Atemluftschlauch 142 führt zu einem allgemein mit 10 bezeichneten Expirationsventil, welches nachfolgend unter Bezugnahme auf die Figuren 2 bis 4 noch näher erläutert werden wird.

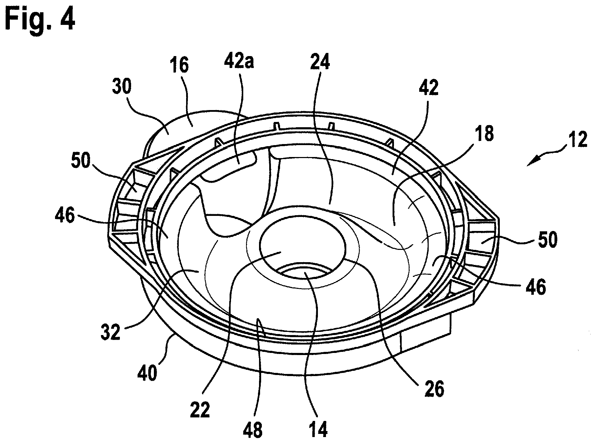

Fig. 2 zeigt in Schnittansicht das in Fig. 1 eingesetzte Expirationsventil 10 gemäß einer Ausführungsform. Das Expirationsventil 10 umfasst einen Ventilkörper 12, in dem ein Fluideinlass 14 und ein Fluidauslass 16 für Atemluft, die von einem Pati - enten ausgeatmet wird, ausgebildet sind. Die Fig. 3 und 4 zeigen perspektivische Ansichten dieses Ventilkörpers 12, und zwar zeigt Fig. 3 eine Ansicht von unten in etwa aus Richtung des mit in Fig. 4 mit 12 bezeichneten Pfeils, und zeigt Fig. 4 eine Ansicht von oben in etwa aus Richtung des in Fig. 3 mit 12 bezeichneten Pfeils. In Fig. 2 ist das Expirationsventil 10 vor dem Einbau in eine Beatmungsmaschine 100 gezeigt, so dass der Atemluftschlauch 142 noch nicht angeschlossen ist. Im betriebsgemäßen Zustand, d.h. nach Einbau des Expirationsventils 10 in eine Beatmungsmaschine 100 gemäß Fig. 1, ist der Atemluftschlauch 142 an dem Fluideinlass 14 angeschlossen. Somit gelangt vom Patienten ausgeatmete Luft über den Fluideinlass 14 zum Expirationsventil 10. Nach Durchgang durch das Expirationsventil 10 gelangt die ausgeatmete Luft über den Fluidauslass 16 in eine Umgebung des Beatmungsgeräts 100.

Das Expirationsventil 10 umfasst ein sogenanntes Ventilplenum 18. Das Ventilple - num 18 bildet einen Hohlraum, der über den Fluideinlass 14 mit Fluid (ausgeatmete Atemluft) beaufschlagbar ist und aus dem über den Fluidauslass 16 Fluid (ausgeatmete Atemluft) ausströmen kann. In dem Ventilplenum 18 ist somit durch den Ventilkörper 12 und eine Ventilmembran 20 ein Fluidströmungsweg zwischen dem Fluideinlass 14 und dem Fluidauslass 16 gebildet. Der Fluideinlass 14 ist so ausgebildet, dass Atemluft beim Ausatmen, d.h. während einer Expirationsphase des Beatmungszyklus, in das Ventilplenum 18 gelangen kann. Während einer In - spirationsphase des Beatmungszyklus, d.h. wenn dem Patienten über die Beatmungsleitung 130 maschinell Atemluft zugeführt wird, soll der Fluideinlass 14 verschlossen sein. Hierfür dient die dem Fluideinlass 14 zugeordnete Ventilmembran 20. Die Ventilmembran 20 ist mindestens zwischen einer den Fluideinlass 14 verschließenden Stellung und einer den Fluideinlass 14 öffnenden Stellung verlager - bar. Die Ventilmembran 20 ist insbesondere so ausgebildet, dass ein zentraler Bereich der Ventilmembran 20 eine vom Fluideinlass 14 gebildete Fluid eintrittsöffnung 22 verschließend am Fluid einlass 14 anliegt, solange der Druck auf der dem Fluideinlass 14 zugeordneten Seite der Ventilmembran 20 nicht um einen vorbestimmten Druck größer ist als auf der auf der vom Fluideinlass 14 abgewandten Seite der Ventilmembran 20 herrschende Druck (das ist normalerweise der Umgebungsdruck). Die Ventilmembran 20 befindet sich dann in einer den Fluideinlass 14 bzw. die Leitung 140 gegenüber dem Ventilplenum 18 verschließenden Stellung. Diese Stellung nimmt die Ventilmembran 20 während der Inspirationsphasen ein. Bei genügend großem Überdruck auf ihrer dem Fluideinlass 14 bzw. der Leitung 140 für ausgeatmete Atemluft zugeordneten Seite gegenüber ihrer vom Fluideinlass 14 bzw. der Leitung 140 für ausgeatmete Atemluft abgewandten Seite bewegt sich die Ventilmembran 20 in eine den Fluideinlass 14 gegenüber dem Ventilplenum 18 freigebende Stellung, so dass Atemluft aus der Leitung 140 für ausgeatmete Atemluft in das Ventilplenum 18 gelangen kann. Dies geschieht üblicherweise während der Expirationsphasen. Die Ventilmembran 20 ist aus einem Kunststoffmaterial hergestellt, insbesondere aus einem Kunststoffmaterial mit elastischen Eigenschaften, beispielsweise einem Silikonkunststoff. Dabei kann die Ventilmembran 20 so eingebaut sein, dass sie ohne Beaufschlagung durch einen Überdruck auf ihrer der Leitung 140 für ausgeatmete Atemluft zugewandten Seite unter Vorspannung an einem die Fluideintrittsöffnung 22 umgebenden Rand 26 des Fluideinlasses 14 anliegt. Bei Beaufschlagung mit einem genügend großen Überdruck auf ihrer der Leitung 140 für ausgeatmete Atemluft zugewandten Seite löst sich die Ventilmembran 20 von dem die Fluideintrittsöffnung 22 umgebenden Rand 26 des Fluideinlasses 14, so dass ausgeatmete Atemluft in das Ventilplenum 18 gelangen kann. Zusätzlich oder alternativ kann aber auch ein Aktor vorgesehen sein, der die Ventilmembran 20 zwischen Verschlussstellung und Freigabestellung hin und her verlagert. Dieser Aktor wird vorzugsweise vom Beatmungsgerät angesteuert und greift beispielsweise in dem zentralen Bereich der Ventilmembran 20 auf der von der Fluid eintrittsöffnung 22 abgewandten Seite der Ventilmembran 20 an. Die jeweilige Stellung der Ventil - membran 20 bestimmt dabei das Volumen des Ventilplenums 18 und den zwischen Ventilmembran 20 und Ventilkörper 12 gebildeten Fluidströmungsweg.

Der Ventilkörper 12 weist einen Ventilboden 24 auf, der sich auf einer Unterseite des Ventilplenums 18 von dem Fluideinlass 14 ausgehend nach außen bis zu ei - nem äußeren Rand des Ventilplenums bzw. Ventilbodens erstreckt. Der Ventilbo - den 24 definiert zusammen mit der Ventilmembran 20 den im Ventilplenum 18 gebildeten Fluidströmungsweg zwischen dem Fluideinlass 14 und dem Fluidauslass 16. Insbesondere bildet der Ventilkörper 12 in bestimmten Ausführungsformen ein topfförmiges Ventilplenum 18, welches wenigstens zum Teil an seiner Unterseite von dem Ventilboden 24 begrenzt ist und in das der Fluideinlass 14 mündet. Der Ventilboden 24 kann sich dabei insbesondere tellerartig zwischen dem Fluideinlass 14 und einem äußeren Rand des Ventilkörpers 12 (insbesondere des Ventilplenums 18) erstrecken, wobei der äußere Rand des Ventilbodens 24 zugleich den äußeren Rand des Ventilkörpers 12 bildet. Der Fluideinlass 14 ist dann insbesondere in einem zentralen Teil des Ventilbodens 24 ausgebildet. Der Fluid einlass 14 kann insbesondere einen kreisförmigen Querschnitt aufweisen mit ebenfalls kreisförmiger Fluideintrittsfläche 22. Ein die Fluideintrittsfläche 22 definierender Rand 26 des Fluideinlasses 14 bildet damit einen inneren Rand des Ventilbodens 24, der sich ausgehend von dem Rand 26 an der Unterseite des Ventilplenums 18 bis zu einem äußeren Rand des Ventilkörpers 12 erstreckt. Der äußere Rand des Ventilkörpers 12 befindet sich in radialer Richtung am weitesten vom Fluideinlass 14 weg. Damit steht der Rand 26 nicht - oder jedenfalls nicht abrupt oder scharfkantig - vom Ventilboden 24 hervor, sondern vielmehr beginnt der Ventilboden 24 unmittelbar am Rand 26 der Fluideintrittsöffnung 22 und erstreckt sich in einer sanft geschwungenen Kontur von diesem Rand 26 aus nach außen. Durch diese Formgebung werden scharfe Kanten vermieden, die bei Umströmung mit Luft zu Strömungsabrissen und damit verbundener Geräuschentwicklung führen könnten.

Der Ventilboden 24 hat eine tellerartige Gestalt. Damit soll zum Ausdruck gebracht werden dass der Ventilboden 24 sich im Wesentlichen flach oder allenfalls flach konisch ausgebildet zwischen seinem inneren Rand 26 und dem äußeren Rand erstreckt. Etwaige Vertiefungen im Ventilboden 24, beispielsweise eine nachfolgend noch näher zu beschreibende Rinne, sind so ausgebildet, dass sich eine insgesamt abgerundete und strömungsangepasste Kontur ergibt mit sanft in - einander übergehenden unterschiedlichen Krümmungen.

Während der Fluideinlass 14 in einem zentralen Bereich des Ventilplenums 18 angeordnet ist, liegt der Fluidauslass 16 bezogen auf den Fluideinlass 14 radial weiter außen. Insbesondere ist der Fluidauslass 16 an einem äußeren Rand des Ventilplenums 18 angeordnet. An der Stelle des Ventilauslasses 16 ist im Ventil - körper 12, insbesondere in einer äußeren Wand des Ventilbodens 24 eine Aus - trittsöffnung ausgebildet durch die Atemluft aus dem Ventilplenum 18 zum Fluid - auslass 16 strömen kann. Auch die Austrittsöffnung ist mit sanft geschwungenen und damit strömungsgünstig ausgebildeten Kanten versehen, so dass ein Ent - langströmen von Atemluft an scharfen Kanten beim Austritt aus dem Ventilplenum 18 vermieden wird.

Der Ventilkörper 12 ist aus Kunststoffmaterial hergestellt, beispielsweise aus Polyethylen, Polypropylen oder einem anderen thermoplastischen Kunststoffmateri - al. Der Ventilkörper 12 ist ein Spritzgussteil, d.h. er ist in einem Spritzgussverfahren hergestellt. Insbesondere kann der Ventilkörper 12 so ausgebildet sein, dass er in einem einzigen Spritzgussprozess herstellbar ist, insbesondere als ein Ein - komponenten-Spritzgussteil.

Der Ventilkörper 12 weist einen Fluideinlassteil 28 auf, in dem der Fluideinlass 14 ausgebildet ist. Der Fluideinlassteil 28 ist insbesondere integral mit dem Ventilkörper 12 ausgebildet. Der Fluideinlassteil 28 erstreckt sich von dem Ventilboden 24 weg, und zwar in Richtung von einer Unterseite des Ventilbodens 24 weg im Wesentlichen entlang einer Achsrichtung A des Fluideinlasses 14. Zudem erstreckt sich der Fluideinlassteil 28 quer zu dem Ventilboden 24, d.h. er steht von einer Unterseite des Ventilbodens 24 in Achsrichtung A des Fluideinlasses 14 vor. Die Achsrichtung A bezeichnet eine Fluideinlassachse, d.h. eine gedachte Linie in Längsrichtung des Fluideinlasssteils 28, die bezüglich des Fluideinlasses 14 zentrat liegt. Bei orthogonal zum Fluideinlass 14 stehender Fluideintrittsfläche 22 steht die Fluideinlassachse A orthogonal zu der durch den Fluideinlass 14 definierten Fluideintrittsfläche 22 und liegt bezüglich der Fluideintrittsfläche 22 zentral.

Der Fluideinlasssteil 28 bildet einen ersten zapfenartigen Ansatz aus, der insbesondere zum Anschluss eines Atemluftschlauches, beispielsweise des in Fig. 1 zu sehenden Atemluftschlauches 142, ausgebildet ist. In der in Fig. 2 gezeigten Variante ist der erste zapfenartige Ansatz insbesondere zylindrisch geformt. In anderen Varianten könnte der zapfenartige Ansatz auch sich zu seinem freien Ende hin leicht verjüngend ausgebildet sein, beispielsweise leicht konisch zulaufend. Der erste zapfenartige Ansatz kann beispielsweise als ein Rohrstutzen ausgebildet sein, in welchem der Fluideinlass 14 ausgebildet ist.

Der Ventilkörper 12 bildet zudem einen Fluidauslassteil 30, in dem der Fluidauslass 16 ausgebildet ist. Der Fluidauslassteil 30 kann insbesondere einen zweiten zapfenartigen Ansatz ausbilden, der zylindrisch oder sich verjüngend (insbeson - dere konisch) geformt sein kann. Der zweite zapfenartige Ansatz kann beispiels - weise als ein zylindrischer Rohrstutzen ausgebildet sein, in welchem der Fluidauslass 16 ausgebildet ist. Auch der Fluidauslassteil 30 kann insbesondere integral mit dem Ventilkörper 12 ausgebildet sein. Der Fluidauslass 16 kann insbesondere einen kreisförmigen Querschnitt aufweisen. Er braucht nicht unbedingt an eine weitere Leitung angeschlossen zu sein, sondern kann direkt in die Umgebung münden, so dass ausgeatmete Luft über den Lufteinlass 16 in die Umgebung abgegeben wird. An seiner radial inneren Seite ist der Fluidauslassteil 16 über die bereits angesprochene Austrittsöffnung mit dem Ventilplenum 18 verbunden, so dass in das Expirationsventil 10 eingeströmtes Fluid über die Austrittsöffnung in den Fluidauslassteil 30 gelangt.

Ähnlich wie der Fluideinlassteil 28 kann sich der Fluidauslassteil 30 insbesondere vom Ventilboden 24 weg erstrecken, insbesondere von einer Unterseite des Ventilbodens 24 weg. Der Fluidauslassteil 30 kann sich orthogonal zu dem Ventilboden 24 erstrecken und/oder in einer Richtung parallel zu dem Fluideinlassteil 28 erstrecken, wie das in den Fig. 2 bis 4 gut zu sehen ist.

Der Fluidströmungsweg in dem Ventilplenum 18 besitzt einen quer zur Fluidströmung zwischen Fluideinlass 14 und Fluidauslass 16 gemessenen Querschnitt, der sich von dem Fluideinlass 14 zum Fluidauslass 16 hin erweitert. Dies wird erreicht durch eine entsprechende Ausbildung und Anordnung des Ventilkörpers 12, insbesondere des Ventilbodens 24, und der Ventilmembran 20. Der Ventilboden 24 bildet vor allem eine bodenseitige Begrenzung des Fluidströmungswegs. Die Ventilmembran 20 bildet vor allem eine Begrenzung des Fluidströmungswegs auf dessen Oberseite. Während durchaus eine Ventilmembran 20 verwendet werden kann, die herkömmlichen Ventilmembranen ähnlich ist, ist der Ventilboden 24 so ausgestaltet, dass sich der Querschnitt des Fluidströmungswegs zum Ventilauslass 16 hin vergrößert. Insbesondere ist der Ventilboden 24 so ausgebildet dass sich sein Abstand zur Ventilmembran 20 vom Fluideinlass 14 her zum Fluideinlass 16 hin vergrößert. Dies kann in einer oder mehreren Stufen geschehen, insbesondere jedoch kontiniuierlich von dem Fluideinlass 14 zu dem Fluidauslass 16 hin. Bei der in Fig. 2 bis 4 zu sehenden Geometrie des Expirationsventils 10 mit radial innen und zentral angeordnetem Fluideinlass 14 und radial außen angeordnetem Fluidauslass 16 bietet es sich beispielsweise an, dass der Fluidströmungsweg so ausgestaltet ist, dass sich auf jeweils gleich große Winkelbereiche um den Fluideinlass 14 herum bezogene Teilvolumina des Fluidströmungswegs entlang des Umfangs des Fluideinlasses 14 verändern, und zwar derart, dass diese Teilvolumina in Richtung zum Fluidauslass 16 hin immer größer werden. Ein solcher Effekt lässt sich beispielsweise erreichen, wenn eine Ebene, entlang der sich der Ventilboden 24 erstreckt, in Bezug auf eine Ebene, entlang der sich die Ventil - membran 20 erstreckt, geneigt ist, und zwar derart, dass diese beiden Ebenen zum Fluidauslass 16 hin auseinander laufen. Während sich beispielsweise die Ventilmembran 20 in aller Regel in einer Ebene orthogonal zu der Fluidein - lassachse A erstrecken wird (in Fig. 2 und 3 ist diese Ebene sehr schematisch durch eine mit C bezeichnete gestrichelte Linie angedeutet), kann in bestimmten Ausführungsformen der Ventilboden 24 sich entlang einer Ebene erstrecken, die gegenüber der Fluideinlassachse A in einem von 90 Grad verschiedenen Winkel geneigt ist. Die Ebene, entlang derer sich der Ventilboden 24 erstreckt, soll dabei die Ebene bezeichnen, die der mittleren Erstreckung des Ventilbodens 24 folgt, wobei von etwaigen einzelnen (etwa dellenförmigen) Erhebungen bzw. Vertiefungen des Ventilbodens 24 abzusehen ist. Besitzt allerdings der Ventilboden 24 eine durchgehend ausgebildete Vertiefung, die den Querschnitt des Fluidströmungswegs zwischen dem Fluideinlass 14 und dem Fluidauslass 16 bestimmt, wie dies nachfolgend noch beschrieben wird, so wird die Ebene, entlang der sich der Ventilboden 24 erstreckt, im Allgemeinen durch den Verlauf dieser Vertiefung (d.h. den Verlauf der tiefsten Stellen in den einzelnen entlang des Fluidströmungswegs aufeinander folgenden Querschnitten) bestimmt werden. In Fig. 2 und 3 ist die Lage dieser Ebene sehr schematisch durch die Linie B angedeutet. Man sieht, dass diese Ebene nicht parallel zu der Ebene ist, entlang der die Ventilmembran 20 verläuft (diese Ebene verläuft orthogonal zu der Fluideinlassachse A), sondern einen von 90 Grad verschiedenen Winkel zu der Fluideinlassachse A bildet. Das impliziert, dass der Normalenvektor N dieser Ebene nicht parallel zu der Fluideinlassachse A ist, sondern einen Winkel α zu dieser bildet. Im Normalfall wird der Normalenvektor N dabei gegenüber der Fluideinlassachse A zu dem dem Fluidauslass 16 hin geneigt sein, wie das auch in Fig. 2 und 3 angedeutet ist.

Die Vergrößerung des Querschnitts des Fluidströmungswegs zum Fluidauslass 16 hin trägt dem Umstand Rechnung, dass dass der Volumenstrom an zum Auslass strömender Atemluft immer mehr zunimmt je näher der Fluidströmungsweg dem Fluidauslass liegt. Das für den Volumentstrom zur Verfügung stehende Volu - men im Ventilplenum 18 ist auf diese Weise an den Volumenstrom angepasst. Man vermeidet durch diese Anpassung des für den Fluidstrom zur Verfügung stehenden Volumens Staueffekte mit daraus resultierender Verwirbelung der auszuatmenden Atemluft.

In Fig. 2 bis 4 ist zudem gut zu sehen, dass der Ventilboden 24 sich in einem Bereich um den Fluideinlass 14 herum in axialer Richtung auswölbt. Mit "in axialer Richtung auswölben" soll hierbei gemeint sein, dass der Ventilboden 24 in wenigstens einem die Fluideinlassachse A enthaltenden Schnitt einen konvexen Verlauf hat, und zwar in einem zwischen dem Rand des Fluideinlasses 14 und dem äußeren Rand des Ventilbodens 24 liegenden Bereich. In den gezeigten Ausführungsformen kann dies ein in radialer Richtung verlaufender Schnitt sein. In Fig. 2 ist dieser konvexe Verlauf für den Schnitt durch die tiefste Stelle der so gebildeten Auswölbung 32 beim Fluidauslass 16 auf der linken Seite in Fig. 2 und für den Schnitt durch die Stelle mit geringster Tiefe der Auswölbung 32 auf der rechten Seite in Fig. 2 angedeutet. Konvex bedeutet, dass in einem solchen Schnitt eine zwei Punkte auf dem Ventilboden 24 verbindende Linie durch das Ventilplenum 18 verläuft. Der Ventilboden 24 bildet demgemäß im Bereich der so entstehenden Auswölbung 32 eine um den Fluideinlass 14 umlaufende Rinne aus, deren Boden sich zwischen dem Rand des Fluideinlasses 14 und dem äußeren Rand des Ventilbodens 24 in axialer Richtung auswölbt. Die Rinne definiert so den um den Fluideinlass 14 herum führenden Fluidströmungsweg. Der entlang des Fluidströmungswegs jeweils tiefste Punkt dieser Rinne (anders ausgedrückt: die Verbindung der in jeweiligen Schnitten zwischen Fluideinlass 14 und äußerem Rand des Ventilbodens 24 tiefsten Punkte) bildet so eine um den Fluideinlass 14 herum führende Linie, die in Fig. 3 mit 34 bezeichnet ist. Bei der in den Figuren gezeigten radialsymmetrischen Geometrie des Ventilbodens 24 mit Fluideinlass 14 in der Mitte ist diese Linie 34 wegen der noch näher zu erläuternden unterschiedlichen Tiefe der Rinne im Wesentlichen ellipsenförmig (die Projektion dieser Linie 34 auf eine zur Fluideinlassachse A orthogonale Ebene, wie die Ebene C, ist kreisförmig). Wie in Fig. 2 bis 4 gut zu sehen ist, bildet der sich auswölbende Bereich des Ventilbodens 24 (oder die so gebildete Rinne) zwei im Wesentlichen um den halben Umfang des Fluideinlasses 14 herum führende Fluidströmungswege aus, die schließlich an einer Seite (in Fig. 2 links vom Fluideinlass 14) in den Fluidauslass 16 münden. Dabei geht der sich auswölbende Bereich des Ventilbodens 24 mit seiner äußeren Seitenwand in den Fluidauslassteil 30 über. Wie bereits angesprochen, haben alle Kanten entlang dieses Fluidströmungswegs eine abgerundete Kontur, die Strömungsabrissen und/oder Verwirbelungen der Fluidströmung möglichst entgegenwirkt.

In den Figuren ist gut zu sehen, dass die axiale Auswölbung 32 des Ventilbodens 24 eine sich um den Umfang des Fluideinlasses 14 herum verändernde Tiefe hat. Die Tiefe ist dabei zu messen als ein in Richtung der Fluideinlassachse A gemessener Abstand zwischen dem Ventilboden 24 am Rand 26 des Fluideinlasses 14 und dem Ventilboden 24 an der tiefsten Stelle der Auswölbung 32 in einem den Fluideinlass 14 mit dem äußeren Rand des Ventilbodens 24 verbindenden Schnitt (bei radialsymmetrischer Geometrie des Ventilbodens 24, wie in Figuren gezeigt, ist das ein radialer Schnitt durch den Ventilboden 24). In Fig. 2 und 3 ist die jeweils größte Tiefe Tmax an der Stelle des Fluidauslasses 16 und die jeweils kleinste Tiefe Tmin an der dem Fluidauslass 16 gegenüber liegenden Seite ange - deutet.

Auf diese Weise verändern sich auf jeweils gleich große Winkelbereiche bezoge - ne Teilvolumina des Fluidströmungswegs entlang des Umfangs des Fluideinlasses 14, und zwar derart, dass diese Teilvolumina in Richtung zum Fluidauslass 16 hin immer größer werden. Es hat sich gezeigt, dass diese Maßnahme ganz erheblich zur Reduzierung von strömungsbedingten Geräuschen beim Abgeben von Atemluft während Expirationsphasen beiträgt, weil das für die Fluidströmung zur Verfügung stehende Volumen dem Volumenstrom des strömenden Fluids an - gepasst ist.

Zudem ist in den Figuren gut zu sehen, dass der Ventilboden 24 ausgehend von einer Innenseite des Fluideinlasses 14 am Rand der Fluideintrittsfläche 22 auf seiner dem Ventilplenum 18 zugewandten Seite eine abgerundete Kontur auf - weist. Dies ist insbesondere in Fig. 4 gut zu erkennen. Der Fluidstrom strömt nach Passieren der Fluideintrittsfläche 22 entlang der abgerundeten inneren Ränder der durch die Auswölbung 32 gebildeten Rinne zu deren tiefster Stelle, um dann entlang des durch die Rinne gebildeten Fluidströmungswegs - etwa der Linie 34 folgend - um den Fluideinlass 14 herum dem Fluidauslass 16 entgegen zu strö - men. Hierbei passiert die Fluidströmung keinerlei scharfe Kanten, die zu Strömungsabrissen oder Verwirbelungen mit damit verursachter Geräuschentwicklung führen würden.

Der Ventilkörper 12 weist außerdem um den Fluideinlass 14 herum ausgebildete Versteifungsrippen 36, 38 auf. In Fig. 2 und 3 sind beispielhaft einige dieser Ver - steifungsrippen mit den Bezugszeichen 36 und 38 bezeichnet. Die Versteifungs - rippen 36, 38 versteifen den Ventilboden 24, insbesondere den durch den Ventil - boden 24 gebildeten Mantel im sich auswölbenden Bereich 32. Verwindungen des Ventilkörpers 12 werden durch diese zusätzliche Versteifung effektiv unter - drückt. Damit tragen die Versteifungsrippen 36, 38 ebenfalls dazu bei, Geräusch - entwicklung zu verringern, weil sie Schwingungen des Ventilbodens 24 unter dem Einfluss von Fluidströmung im Ventilplenum 18 unterdrücken. Damit lassen sich aber auch ungewollte störende Schwingungen der Ventilmembran 20 effektiv un - terdrücken. Dadurch sinkt die Gefahr für Oszillationen der Luftsäule im Leitungs - system, welche zu fälschlich ausgelösten Steuervorgängen (insbesondere soge - nannte Autotrigger) führen können. Die Versteifungsrippen 36, 38 sind integral mit dem Ventilkörper 12 ausgebildet. Integral ist dabei so verstehen, dass die Verstei - fungsrippen 26 28 und der Ventilkörper 12 als ein einziges Bauteil hergestellt sind. In dem gezeigten Ausführungsbeispiel sind die Versteifungsrippen 36, 38 im selben Spritzgussprozess hergestellt wie der Ventilkörper 12.

Die Versteifungsrippen 36, 38 sind außerhalb des Fluideinlasses 14 angeordnet. Wie in den Figuren zu sehen ist, verlaufen sie bezogen auf den Rand 26 des Fluideinlasses 14 nach außen zum Rand des Ventilkörpers 12 hin. In dem gezeigten Ausführungsbeispiel verlaufen die Versteifungsrippen bezüglich der Fluidein - lassachse A in radialer Richtung. Sie könnten aber auch eher spiralförmig von innen nach außen führen. Die Versteifungsrippen verbinden dabei den Rand 26 des Fluideinlasses 14 mit dem äußeren Rand des Ventilkörpers 12. Die Versteifungsrippen 36, 38 sind auf einer Unterseite des Ventilbodens 24 ausgebildet. Somit stören sie den auf der Innenseite des Ventilkörpers 12, d.h. an der Obersei - te des Ventilbodens 24 ausgebildeten Fluidströmungsweg nicht. Wie in den Figu - ren zu sehen ist, umfassen die Versteifungsrippen erste Versteifungsrippen 36 und zweite Versteifungsrippen 38. Die ersten Versteifungsrippen 36 sind innere Versteifungsrippen, die zwischen dem Rand 26 des Fluideinlasses 14 und dem sich auswölbenden Bereich 32 des Ventilbodens 24 verlaufen. In dem gezeigten Ausführungsbeispiel verbinden die ersten Versteifungsrippen 36 den Rand 26 des Fluideinlasses 14 mit dem sich auswölbenden Bereich 32 des Ventilbodens 24. Die zweiten Versteifungsrippen 38 sind äußere Versteifungsrippen, die zwi - schen dem sich auswölbenden Bereich des Ventilbodens 32 und dem äußeren Rand des Ventilbodens 24 verlaufen. In dem gezeigten Ausführungsbeispiel ver - binden die zweiten Versteifungsrippen 38 den sich auswölbenden Bereich 32 des Ventilbodens 24 mit dem äußeren Rand des Ventilbodens 24. Die zweiten Ver - steifungsrippen 38 enden in einem am äußeren Rand des Ventilbodens 24 aus - gebildeten flanschartigen Ansatz 40. Der Ansatz 40 kann beispielsweise mindes - tens einen um den äußeren Rand des Ventilbodens 24 umlaufenden Absatz auf - weisen, insbesondere mindestens zwei im Abstand zueinander angeordnete Ab - sätze, wobei die zweiten Versteifungsrippen 38 in den Absatz, bzw. einen inneren der Absätze, übergehen. Der Absatz bzw. die Absätze kann beispielsweise einen ringartigen Flansch bilden, der um den äußeren Rand des Ventilbodens 24 um - läuft und diesem Stabilität verleiht.

Wie in Fig. 2 gezeigt ist, weist das Expirationsventil 10 ferner die dem Fluidein - lass 14 zugeordnete Ventilmembran 20 auf. Die Ventilmembran 20 liegt im in das Expirationsventil 10 eingebauten Zustand mit einer an ihrem äußeren Rand ausgebildeten Anlagefläche an der Oberseite einer Auflage 42 an, die an der Ober - seite des Ventilkörpers 12 ausgebildet ist. Die Auflage 42 bildet einen um den äu - ßeren Rand des Ventilkörpers 12 umlaufenden Vorsprung, der von dem äußeren Rand des Ventilkörpers 12 nach innen, zur Seite des Ventilplenums 18, vorspringt. Die Auflage 42 ist ebenfalls integral mit dem Ventilkörper 12 ausgebildet, beispielsweise als Spritzgussteil im selben Spritzgussprozess ausgebildet wie der Ventilkörper 12. Beim Zusammenbau des Expirationsventils 10 wird die Ventilmembran 20 mit ihrer äußeren Anlagefläche in Anlage gebracht mit der Oberseite der Auflage 42 des Ventilkörpers 12, so dass die Ventilmembran 20 mit einer an ihrem zentralen Teil ausgebildeten Anlagefläche auf dem Rand 26 des Fluidein - lasses 14 aufliegt und mit der an ihrem äußeren Teil ausgebildeten Anlagefläche an der Auflage 42 aufliegt. Die Ventilmembran 20 bildet so eine Oberseite des im Ventilplenum 18 gebildeten Fluidströmungswegs. Die Ventilmembran 20 wird dann mittels eines in Fig. 2 gezeigten Spannrings 44 gegenüber dem Ventilkörper 12 eingespannt.

Die Auflage 42 kann um den Umfang des äußeren Randes des Ventilkörpers um - laufend ausgebildet sein (wie etwa in dem mit 42 bezeichneten Bereich in Fig. 4) oder mit Unterbrechungen ausgebildet sein, so dass sich einzelnen Auflageberei - che ergeben, die entlang des Umfangs des äußeren Randes des Ventilkörpers 12 an aufeinander folgen. In dem Ausführungsbeispiel gem. Fig. 4 ist ein solcher ein - zelner Auflagebereich 42a auf der Seite des Fluideinlasses 16 oberhalb der Aus - trittsöffnung zwischen dem Ventilplenum 18 und dem Fluidauslass 16 ausgebil - det.

Die Oberseite 46 der Auflage 42 ist uneben ausgebildet, so dass die Oberseite 46 der Auflage 42 in einer gedachten gewölbten Fläche liegt, es also es keine ge - dachte Ebene gibt, die die Oberseite 46 der Auflage 42 vollständig enthält. Die gedachte gewölbte Fläche überdeckt eine Oberseite des Ventilkörpers 12. Sie liegt insbesondere orthogonal zu der Fluideinlassachse A, die zugleich eine Längsachse des Ventilkörpers 12 bildet. Wegen der unebenen Form der Obersei - te 46 der Auflage 42 würde eine ebene Fläche, die man auf die Oberseite 46 der Auflage aufzulegen versucht, nicht die gesamte Oberseite 46 der Auflage 42 be - rühren. Vielmehr würden nur einzelne Stellen der ebenen Fläche Kontakt mit der Oberseite 46 der Auflage 42 haben. Daher liegt, jedenfalls im nicht in ein Beat - mungsgerät 100 eingebauten Zustand des Expirationsventils 10 die ebene Ventil - membran 20 nicht entlang ihres gesamten Umfangs an der Oberseite 46 der Auf - lage 42 auf. Es gibt vielmehr einzelne Berührungsstellen zwischen Ventilmem - bran 20 und Auflage entlang des Umfangs der Ventilmembran 20. Die Unebenheit der Oberseite der Auflage 42 ist nun so ausgebildet, dass erst dann, wenn das Expirationsventil 10 in das Beatmungsgerät 100 eingebaut ist und der Ventilkörper 12 unter Spannung steht, so dass er sich deformiert, die Oberseite 46 der Auflage 42 in einer ebenen Fläche zu liegen kommt und die Ventilmembran 20 nun mit ihrer Anlagefläche flächig auf der Oberseite 46 der Auflage 42 aufliegt.

Der Ventilkörper 12 weist wenigstens eine Montagelasche 50 auf, die zum Eingriff mit einem bajonettartigen Expirationsventilanschluss eines Beatmungsgeräts (100) ausgebildet ist. Das Beatmungsgerät weist dann ein Gegeneingriffselement auf, mit dem die Montagelasche 50 beim Einbau des Expirationsventils 10 in Ein - griff gelangt, um das Expirationsventil 10 am Beatmungsgerät 100 zu halten. Ein solcher Bajonettverschluss hat die Eigenschaft, dass beim Verriegeln des Expira - tionsventils 10 am Beatmungsgerät 100 Spannkräfte entstehen, die den Ventilkörper 12 etwas deformieren.

Der Ventilkörper 12 weist wenigstens zwei Montagelaschen 50 auf, die einander zugeordnet sind. Die Montagelaschen 50 sind am radial äußeren Rand des Ven - tilkörpers 12 um einen Umfang des Ventilkörpers 12 herum angeordnet und stehen in radialer Richtung vom jeweiligen radial äußeren Rand des Ventilkörpers 12 hervor, um jeweilige Eingriffsflächen 52 mit einem entsprechenden Gegenein - griffselement am Beatmungsgerät 100 zu bilden. Bringt man die Montagelaschen 50 in Eingriff mit ihren jeweiligen am Beatmungsgerät 100 vorgesehenen Gegen - eingriffselementen, wird der Ventilkörper 12 auf den jeweils zugeordneten Seiten, an denen sich die Montagelaschen 50 befinden, zum Beatmungsgerät 100 hin geführt. Im Zuge dieser Zwangsbewegung entsteht eine Spannung im Ventilkörper 12, und die obere Kante 48 des Ventilkörpers 12 biegt sich auf den Seiten, an denen die Montagelaschen 50 angeordnet sind, zum Beatmungsgerät 100 hin. Die unebene Ausgestaltung der Oberseite 46 der am Ventilkörper 12 vorgesehe - nen Auflage 42 für die Ventilmembran 20 soll diese Verbiegung des Ventilkörpers 12 ausgleichen. Nach Ineingriffbringen der Montagelaschen 50 mit dem jeweili - gen Gegeneingriffselement am Beatmungsgerät 100 soll die Oberseite 46 der Auflage 42 flach sein, also in einer Ebene liegen. Das beiden Montagelaschen 50 liegen einander in einem Winkel von 180° zueinander gegenüber, d.h. auf gegen - überliegenden Seiten des Ventilkörpers 12 über das Ventilplenum 18 hinweg.

Die Oberseite 46 der Auflage 42 liegt in einer sich bezogen auf das Ventilplenum 18 nach außen gewölbten gedachten Fläche, die die Oberseite des Ventilkörpers 12 überdeckt. Insbesondere liegt die nach außen gewölbte gedachte Fläche be - zogen das Ventilplenum 18 weiter außen als eine gedachte Ebene durch wenigs - tens zwei verschiedene Punkte auf der Oberseite 46 der Auflage 42 an Seiten, an denen Montagelaschen 50 vorhanden sind (also auf der linken und rechten Seite in Fig. 4). Die Oberseite 46 der Auflage 42 liegt dabei in einer konvex gewölbten gedachten Fläche, so dass eine zwei in der gedachten Fläche liegende Punkte verbindende Linie durch das Ventilplenum 18 verläuft. Dabei wölbt sich die Ober - seite 46 der Auflage 42 ausgehend von den beiden Montagelaschen 50 nach au - ßen. An den Stellen, an denen sich die Montagelaschen 50 befinden, wölbt sich die Oberseite 46 der Auflage 42 gar nicht nach außen, wohingegen sie sich in der Mitte zwischen den beiden Montagelaschen 50 (in Fig. 4 entlang einer Linie von oben nach unten orthogonal zu einer Verbindung zwischen den beiden Montage - laschen 50) am meisten nach außen auswölbt. Dies trägt der Tatsache Rechnung, dass die Deformierung des Ventilkörpers 12 beim Befestigen des Expirati - onsventils 10 am Beatmungsgerät 100 dort am stärksten ist, wo sich die Montagelaschen 50 befinden. An dieser Stelle wird die obere Kante 48 des Ventilkör - pers 12 am stärksten nach oben gebogen.

In dem gezeigten Ausführungsbeispiel ist die gewölbte gedachte Fläche eine Mantelfläche eines Zylinders, wobei die Achse des Zylinders orthogonal zu einer Verbindungslinie zwischen den beiden zugeordneten Montagelaschen 50 ist.

Wegen der beim Befestigen des Expirationsventils 10 an Stellen, an den Monta - gelaschen 50 sind, auf den den Ventilkörper 12 ausgeübten Spannung biegt sich auch die obere Kante 48 des Ventilkörpers 12 in derselben Weise an diesen Stel - len zu einer Gegenseite am Beatmungsgerät 100 hin. Zur Kompensation dieses Effektes ist es günstig, auch den Ventilkörper 12, genauer gesagt, die obere Kante 48 des Ventilkörpers 12, in entsprechender Weise auszubilden wie die Auflage 42 für die Ventilmembran 20. Die obere Kante 48 des Ventilkörpers soll 12 nach Befestigen des Expirationsventils 10 am Beatmungsgerät 100 an einer Gegenflä - che des Beatmungsgeräts 100 anliegen und zwar in einer möglichst flächigen Weise, um eine möglichst dichte Anlage der Ventilmembran 20 zu gewährleisten. Deshalb weist der Ventilkörper 12 eine obere Kante 48 auf, welche in einer sich bezogen auf das Ventilplenum 18 nach außen gewölbten gedachten Fläche liegt. Die bereits dargelegten Überlegungen für die Oberseite 46 der Auflage 42 gelten für die Ausgestaltung der oberen Kante 48 des Ventilkörpers 12 in entsprechen - der Weise. Insbesondere liegt die obere Kante 48 des Ventilkörpers 12 in einer sich bezogen auf das Ventilplenum 18 konvex gewölbten gedachten Fläche und wölbt sich ausgehend von der wenigstens einen Montagelasche 50 aus nach au - ßen. Dabei ist die Auswölbung der oberen Kante 48 des Ventilkörpers 12 in einem zwischen den beiden Montagelaschen 50 liegenden Bereich größer als in einem einer der beiden Montagelaschen 50 benachbarten Bereich.

Die unebene Form der Oberseite 46 der Auflage 42 und die unebene Form der oberen Kante 48 des Ventilkörpers 12 sind aufeinander abgestimmt. Das ist sinn - voll, denn es soll für beide erreicht werden, dass nach Befestigen des Expirati - onsventils 10 am Beatmungsgerät 100 sowohl die Oberseite 46 der Auflage 42 als auch die obere Kante 48 des Ventilkörpers 12 in einer ebenen Fläche liegt. Insbesondere wölben sich die Oberseite 46 der Auflage 42 und die obere Kante 48 des Ventilkörpers 12 zueinander ausgerichtet nach außen, wobei die Orte mi - nimaler Auswölbung und maximaler Auswölbung aufeinander abgestimmt sind, insbesondere in Bezug auf eine das Ventilplenum 18 überdeckende Ebene an derselben Stelle liegen. Es ist sogar so, dass die Oberseite 46 der Auflage 42 und die obere Kante 48 des Ventilkörpers 12 sich in etwa gleichermaßen auswölben und insbesondere in in zueinander parallelen gedachten Flächen liegen.

Jeder der Montagelaschen 50 weist eine Eingriffsfläche 52 zum Eingriff mit einem am Beatmungsgerät 100 vorgesehenen Gegeneingriffselement auf, wobei die Eingriffsfläche 52 mit einer Einlaufschräge 52a versehen ist, die zu der Auswöl - bung der Oberseite der Anlage 42 abgestimmt ist. Wie bei einem Bajonetteingriff üblich, wird die Eingriffsfläche 52 in der Regel im Wesentlichen parallel zu einer oberen Kante der Montagelasche ausgebildet sein. In ihrem vorderen Bereich ist die Einlaufschräge 52a vorgesehen. Im Bereich der Einlaufschräge 52a verläuft die Eingriffsfläche 52 dann in einem Winkel, insbesondere einem spitzen Winkel, zu der oberen Kante der Montagelasche 50. Der Winkel ist so gewählt, dass eine Dicke zwischen Eingriffsfläche 52 und oberer Kante der Montagelasche 50 mit zunehmendem Abstand vom Beginn der Eingriffsfläche 52a zunimmt. Damit lässt sich eine gewisse Spannung beim Ineingriffbringen der Montagelasche 50 mit dem Gegeneingriffselement am Beatmungsgerät 100 erzeugen, was zu einem gewissen Kraftschluss und damit einem festen und sicheren Sitz des Expirations - ventils 10 am Beatmungsgerät 100 verhilft. Die Einlaufschräge 52a ist so ausge - bildet, dass beim Einspannen der Montagelasche 50 am Beatmungsgerät 100 eine Vorspannung erzeugt wird, die auf die obere Kante 48 des Ventilkörpers auf der Seite der Montagelasche 50 einwirkt. Damit einher geht eine gewisse Deformation des Ventilkörpers 12 auf der der Montagelasche 50 zugewandten Seite gegenüber Seiten oder Stellen weiter abseits der Montagelasche 50. Insbesonde - re wird die obere Kante 48 des Ventilkörpers 12 durch Bewegung des Gegenein - griffselements entlang der Einlaufschräge 52a näher zum Beatmungsgerät 100 hin geführt. Andererseits liegen weiter von der Montagelasche 50 entfernte Stel - len bereits an einer Kontaktfläche des Beatmungsgeräts 100 an und können sich nicht weiter annähern. Im Ergebnis entsteht eine Verbiegung der oberen Kante 48 des Ventilkörpers 12, wobei die Verbiegung zu der Montagelasche 50 hin immer stärker wird. Dadurch entsteht eine Vorspannung im Ventilkörper 12, die mit zunehmender Bewegung des Gegeneingriffselements entlang der Einlaufschräge 52a zunimmt. Die Einlaufschräge 52a ist so gewählt, dass beim Einspannen eine Vorspannung erzeugt wird, die gerade groß genug ist, um die obere Kante 48 des Ventilkörpers 12 im eingespannten Zustand eben zu machen, d.h. dass die obere Kante 48 des Ventilkörpers 12 im eingespannten Zustand in einer das Ventilple - num 18 überdeckenden Ebene liegt. Dann liegt die obere Kante 48 des Ventilkör - pers 12 flächig an einer Gegenfläche des Beatmungsgeräts 100 an. Gleicherma - ßen verbiegt sich auch die integral mit dem Ventilkörper 12 ausgebildete Auflage 42 für die Ventilmembran 20. Damit die Oberseite 46 dieser Auflage 42 im am Beatmungsgerät 100 befestigten Zustand des Expirationsventils 10 ebenfalls eben ist, ist es günstig, wenn die Oberseite 46 der Auflage 42 in demselben Maße un - eben ist wie die obere Kante 48 des Ventilkörpers 12. Die Einlaufschräge 52a der Montagelasche 50 ist demgemäß auf die Auswölbung der Auflage 42 abgestimmt.

Um das Ende der Einlaufschräge 52a möglichst gut zu definieren und damit die Vorspannung für den Ventilkörper 12 möglichst genau einstellen zu können, ist vorgesehen sein, dass die Eingriffsfläche 52 einen auf die Einlaufschräge 52a fol - genden nicht abgeschrägten Bereich 52b aufweist. Im nicht abgeschrägten Be - reich 52b ist die Eingriffsfläche 52 parallel zu der oberen Kante der Montagela - sehe 50. Eine einmal erreichte Vorspannung und Verbiegung des Ventilkörpers 12 änderst sich damit nicht mehr, wenn das Gegeneingriffselement den nicht abge - schrägten Bereich 52b erreicht. Damit kann die Vorspannung und Verbiegung gut reproduzierbar eingestellt werden, weil es nicht genau darauf ankommt, bis zu welcher Stelle des nicht abgeschrägten Bereichs 52b die Montagelasche 50 ge - langt. Zudem ist noch die Eingriffsfläche 52 an ihrem Ende mit einem Anschlag 54 versehen, der ein Ende der Ineingriffbewegung zwischen Montagelaschen 50 und Beatmungsgerät 100 festlegt.

Das beschriebene Expirationsventil 10 kann als ein Einwegbauteil ausgebildet sein, d.h. so ausgebildet sein, dass es nur einmalig einsetzbar ist und nach Ge - brauch entsorgt werden muss. Solche Expirationsventile sind billig herzustellen und erfüllen doch die geforderten Hygienestandards. Alternativ kann das Expirati - onsventil 10 auch so ausgebildet sein, dass ein mehrmalige Verwendung möglich ist. Hierfür wäre die Gestaltung des Expirationsventils 10 so auszuführen, dass es nach Gebrauch desinfizierbar ist, beispielsweise durch Autoklavieren. Das Expira - tionsventil ist dann autoklavierfest auszulegen.

## Patentansprüche

1. Expirationsventil (10) eines Beatmungsgeräts (100), aufweisend
einen Ventilkörper (12), in dem ein Fluideinlass (14) und ein Fluidauslass (16) für Atemluft eines Patienten ausgebildet sind, wobei der Ventilkörper (12) eine Auflage (42) aufweist mit einer Oberseite (46), an der eine Ventilmembran (20) mit einer an ihrem äußeren Rand ausgebildeten Anlagefläche auflegbar ist, wobei die Oberseite (46) der Auflage (42) uneben ausgebildet ist;
**dadurch gekennzeichnet, dass**
die Oberseite (46) der Auflage (42) in einer bezogen auf ein vom Ventilkörper (12) umgebenes Ventilplenum (18) nach außen gewölbten gedachten Fläche liegt,
und die Unebenheit der Oberseite der Auflage (42) so ausgebildet ist, dass erst dann, wenn das Expirationsventil (10) in das Beatmungsgerät (100) eingebaut ist und der Ventilkörper (12) unter Spannung steht, so dass er sich deformiert, die Oberseite (46) der Auflage (42) in einer ebenen Fläche zu liegen kommt und die Ventilmembran (20) nun mit ihrer Anlagefläche flächig auf der Oberseite (46) der Auflage (42) aufliegt.

2. Expirationsventil (10) nach Anspruch 1, ferner aufweisend eine dem Fluideinlass (14) zugeordnete Ventilmembran (20), die mindestens zwischen einer den Fluideinlass (14) verschließenden Stellung und einer den Fluideinlass (14) öffnenden Stellung verlagerbar ist, wobei die Ventilmembran (20) mit einer an ihrem äußeren Rand ausgebildeten Anlagefläche auf der der Oberseite der Auflage (42) aufliegt.

3. Expirationsventil (10) nach Anspruch 2, ferner aufweisend ein Spannelement (44), insbesondere einen Spannring, welches mit dem Ventilkörper (12) derart zusammenwirkt, dass das Spannelement (44) am Außenumfang der Ventilmembran (20) in Spanneingriff mit der Ventilmembran (20) gelangt.

4. Expirationsventil (10) nach einem der Ansprüche 1 bis 3, wobei der Ventilkörper (12) wenigstens eine Montagelasche (50) aufweist, die zum Eingriff mit einem Expirationsventilanschluss eines Beatmungsgeräts (100) ausgebildet ist.

5. Expirationsventil (10) nach Anspruch 4, wobei der Ventilkörper (12) wenigstens zwei Montagelaschen (50) aufweist, die einander zugeordnet sind.

6. Expirationsventil (10) nach einem der Ansprüche 1 bis 5, wobei die Oberseite (46) der Auflage (42) in einer konvex gewölbten gedachten Fläche liegt.

7. Expirationsventil (10) nach einem der Ansprüche 4 bis 6, wobei die Oberseite (46) der Auflage (42) sich ausgehend von der wenigstens einen Montagelasche (50) nach außen auswölbt.

8. Expirationsventil nach einem der Ansprüche 5 bis 7, wobei die Auswölbung der Auflage (42) in einem zwischen den Montagelaschen (50) liegenden Bereich am größten ist.

9. Expirationsventil nach Anspruch 8, wobei die Auswölbung der Auflage (42) entlang einer zwischen den beiden Montagelaschen (50) verlaufenden Linie am größten ist.

10. Expirationsventil (10) nach einem der Ansprüche 6 bis 9, wobei die gewölbte gedachte Fläche eine Mantelfläche eines Zylinders ist.

11. Expirationsventil (10) nach einem der Ansprüche 1 bis 10, wobei der Ventilkörper (12) eine obere Kante (48) aufweist, welche in einer sich bezogen auf ein vom Ventilkörper (12) umgebenes Ventilplenum (18) nach außen gewölbten gedachten Fläche liegt.

12. Expirationsventil (10) nach Anspruch 11, wobei die obere Kante (48) des Ventilkörpers (48) in einer sich bezogen auf ein vom Ventilkörper (12) umgebenes Ventilplenum (18) konvex gewölbten gedachten Fläche liegt.

13. Expirationsventil (10) nach Anspruch 11 oder 12, wobei die obere Kante (48) des Ventilkörpers (12) sich ausgehend von der wenigstens einen Montagelasche (50) auswölbt.

14. Expirationsventil (10) nach einem der Ansprüche 11 bis 13, wobei die Auswölbung der oberen Kante (48) des Ventilkörpers (12) in einem zwischen den beiden Montagelaschen (50) liegenden Bereich am größten ist.

15. Expirationsventil (10) nach Anspruch 14, wobei die Auswölbung der oberen Kante (48) des Ventilkörpers (12) entlang einer zwischen den beiden Montagelaschen (50) verlaufenden Linie am größten ist.

16. Expirationsventil (10) nach einem der Ansprüche 12 bis 15, wobei die gewölbte gedachte Fläche eine Mantelfläche eines Zylinders ist.

17. Expirationsventil nach einem der Ansprüche 11 bis 16, wobei die Oberseite (46) der Auflage (42) und die obere Kante (48) des Ventilkörpers (12) sich zueinander ausgerichtet nach außen wölben.

18. Expirationsventil nach Anspruch 17, wobei die Oberseite (46) der Auflage (42) und die obere Kante (48) des Ventilkörpers (12) in zueinander parallelen gedachten Flächen liegen.

19. Expirationsventil (10) nach einem der Ansprüche 3 bis 18, wobei die Montagelasche (50) eine Eingriffsfläche (52) zum Eingriff mit einem am Beatmungsgerät (100) vorgesehenen Gegeneingriffselement aufweist, wobei die Eingriffsfläche (52) mit einer Einlaufschräge (52a) versehen ist, die zu der Auswölbung der Oberseite (46) der Anlage (42) abgestimmt ist.

20. Expirationsventil (10) nach Anspruch 19, wobei die Eingriffsfläche (52) einen auf die Einlaufschräge (52a) folgenden nicht abgeschrägten Bereich (52b) aufweist.

21. Expirationsventil nach Anspruch 19 oder 20, wobei die Eingriffsfläche (52) an ihrem Ende mit einem Anschlag (54) versehen ist.

22. Expirationsventil (10) nach einem der Ansprüche 1 bis 21, wobei der Ventilkörper (12) aus Kunststoffmaterial hergestellt ist.

23. Expirationsventil (10) nach Anspruch 22, wobei der Ventilkörper (12) ein Spritzgussteil ist.

24. Expirationsventil (10) nach einem der Ansprüche 1 bis 23, wobei der Ventilkörper (12) ein topfförmiges Ventilplenum (18) bildet, in das der Fluideinlass (14) mündet.

25. Expirationsventil (10) nach Anspruch 24, wobei der Fluideinlass (14) in einem zentralen Bereich des Ventilplenums (18) angeordnet ist.

26. Expirationsventil (10) nach Anspruch 24 oder 25, wobei der Ventilkörper (12) einen das Ventilplenum (18) wenigstens zum Teil einschließenden Ventilboden (24) bildet, welcher sich tellerartig zwischen dem Fluideinlass (14) und einem Außenrand des Ventilplenums (18) erstreckt.

27. Expirationsventil (10) nach Anspruch 26, wobei der Ventilboden (24) einen Fluidströmungsweg zwischen dem Fluideingang (14) und dem Fluidausgang (16) definiert, dessen Querschnitt sich zum Fluidausgang (16) hin erweitert.

28. Expirationsventil (10) nach Anspruch 26 oder 27, wobei der Ventilboden (24) sich in einem Bereich um den Fluideinlass (14) herum in axialer Richtung auswölbt.

29. Expirationsventil (10) nach Anspruch 28, ferner aufweisend eine axiale Auswölbung (32) mit einer sich um den Umfang des Fluideinlasses (14) herum zu dem Fluidauslass (16) hin zunehmenden Tiefe.

30. Expirationsventil nach einem der Ansprüche 1 bis 29, wobei der Ventilkörper (12) wenigstens eine um den Fluideinlass (14) herum ausgebildete Versteifungsrippe (36, 38) aufweist.

31. Beatmungsgerät, aufweisend ein an eine Expirationsluftleitung anschließbares Expirationsventil (10) nach einem der vorangehenden Ansprüche.

32. Verfahren zum Zusammenbau eines Expirationsventils (10) mit einem Beatmungsgerät (100), bei welchem eine Ventilmembran (20) mit einer entlang ihres Außenumfangs ausgebildeten Anlagefläche in Anlage gebracht wird mit einer Oberseite (46) einer an einem Ventilkörper (12) des Expirationsventils (10) ausgebildeten Auflage (42) und das Expirationsventil (10) an dem Beatmungsgerät (100) befestigt wird, wobei der Ventilkörper (12) durch Spannkräfte verformt wird, wobei die Oberfläche (46) der am Ventilkörper (12) ausgebildeten Auflage (42) in einer solchen Weise uneben ausgebildet ist, dass die beim Befestigen des Expirationsventils (10) am Beatmungsgerät (100) durch Spannkräfte erzeugte Verformung des Ventilkörpers (12) dazu führt, dass die Oberseite (46) der Auflage (42) eben wird;
wobei die Oberseite (46) der Auflage (42) in einer sich bezogen auf ein vom Ventilkörper (12) umgebenes Ventilplenum (18) nach außen gewölbten gedachten Fläche liegt.

## Claims

1. An expiratory valve (10) of a ventilation device (100), comprising
a valve body (12) having a fluid inlet (14) and a fluid outlet (16) for breathing air of a patient formed therein, said valve body (12) comprising a support (42) having a top side (46) on which a valve membrane (20) can be placed with an abutment area formed at the outer edge thereof, with the top side (46) of the support (42) having an uneven design;
**characterized in that**
the top side (46) of the support (42) is located in an outwardly curved imaginary surface with respect to the valve plenum (18) enclosed by the valve body (12), and
that the unevenness of the top side of the support (42) is such that, only when the expiratory valve (10) is installed in the ventilation device (100) and the valve body (12) is subject to stress causing deformation of the same, the top side (46) of the support (42) comes to lie in a planar surface and the valve membrane (29) now has its abutment area in sheet-like contact with the top side (46) of the support (42).

2. The expiratory valve (10) of claim 1,
further comprising a valve membrane (20) associated with the fluid inlet (14), which is displaceable at least between a position closing the fluid inlet (14) and a position opening the fluid inlet (14), with the valve membrane (20) resting on the top side of the support (42) with an abutment area formed at the outer edge thereof.

3. The expiratory valve (10) of claim 2,
further comprising a clamping member (44), in particular a clamping ring, cooperating with the valve body (12) such that the clamping member (44) establishes clamping engagement with the valve membrane (20) at the outer periphery of the valve membrane (20).

4. The expiratory valve (10) of any of claims 1 to 3,
wherein the valve body (12) comprises at least one mounting lug (50) which is designed for engagement with an expiratory valve connection of a ventila tion device (100).

5. The expiratory valve (10) of claim 4,
wherein the valve body (12) comprises at least two mounting lugs (50) associated with each other.

6. The expiratory valve (10) of any of claims 1 to 5,
wherein the top side (46) of the support (42) is located in a convexly curved imaginary surface.

7. The expiratory valve (10) of any of claims 4 to 6,
wherein the top side (46) of the support (42) bulges outwardly starting from the at least one mounting lug (50).

8. The expiratory valve (10) of any of claims 5 to 7,
wherein the bulge of the support (42) is largest in a region located between the mounting lugs (50).

9. The expiratory valve of claim 8,
wherein the bulge of the support (42) is largest along a line extending be tween the two mounting lugs (50).

10. The expiratory valve (10) of any of claims 6 to 9,
wherein the curved imaginary surface is a lateral surface of a cylinder.

11. The expiratory valve (10) of any of claims 1 to 10,
wherein the valve body (12) has an upper edge (48) which is located in an outwardly curved imaginary surface with respect to a valve plenum (18) enclosed by the valve body (12).

12. The expiratory valve (10) of claim 11,
wherein the upper edge (48) of the valve body (12) is located in a convexly curved imaginary surface with respect to a valve plenum (18) enclosed by the valve body (12).

13. The expiratory valve (10) of claim 11 or 12,
wherein the upper edge (48) of the valve body (12) bulges starting from the at least one mounting lug (50).

14. The expiratory valve (10) of any of claims 11 to 13,
wherein the bulge of the upper edge (48) of the valve body (12) is largest in a region located between the two mounting lugs (50).

15. The expiratory valve (10) of claim 14,
wherein the bulge of the upper edge (48) of the valve body (12) is largest along a line extending between the two mounting lugs (50).

16. The expiratory valve (10) of any of claims 12 to 15,
wherein the curved imaginary surface is a lateral surface of a cylinder.

17. The expiratory valve of any of claims 11 to 16,
wherein the top side (46) of the support (42) and the upper edge (48) of the valve body (12) bulge outwardly in mutually aligned manner.

18. The expiratory valve of claim 17,
wherein the top side (46) of the support (42) and the upper edge (48) of the valve body (12) are arranged in mutually parallel imaginary surfaces.

19. The expiratory valve (10) of any of claims 3 to 18,
wherein the mounting lug (50) has an engagement surface (52) for engage ment with a mating engagement member provided at the ventilation device (100), the engagement surface (52) being provided with a lead-in chamfer (52a) that is matched to the bulge of the top side (46) of the support (42).

20. The expiratory valve (10) of claim 19,
wherein the engagement surface (52) comprises a non-tapering portion (52b) following the lead-in chamfer (52a).

21. The expiratory valve of claim 19 or 20,
wherein the engagement surface (52) is provided with a stop (54) at the end thereof.

22. The expiratory valve (10) of any of claims 1 to 21,
wherein the valve body (12) is made of plastics material.

23. The expiratory valve (10) of claim 22,
wherein the valve body (12) is an injection-molded part.

24. The expiratory valve (10) of any of claims 1 to 23,
wherein the valve body (12) forms a cup-shaped valve plenum (18) into which the fluid inlet (14) opens.

25. The expiratory valve (10) of claim 24,
wherein the fluid inlet (14) is disposed in a central portion of the valve plenum (18).

26. The expiratory valve (10) of claim 24 or 25,
wherein the valve body (12) forms a valve bottom (24) which at least partly encloses the valve plenum (18), said valve bottom (24) extending in dish-like manner between the fluid inlet (14) and an outer edge of the valve plenum (18).

27. The expiratory valve (10) of claim 26,
wherein the valve bottom (24) defines a fluid flow path between the fluid inlet (14) and the fluid outlet (16), with the cross-sectional area of the fluid flow path expanding in the direction towards the fluid outlet (16).

28. The expiratory valve (10) of claim 26 or 27,
wherein the valve bottom (24) bulges in axial direction in a portion around the fluid inlet (14).

29. The expiratory valve (10) of claim 28,
further comprising an axial bulge (32) having a depth increasing around the circumference of the fluid inlet (14) towards the fluid outlet (16).

30. The expiratory valve (10) of any of claims 1 to 29,
wherein the valve body (12) comprises at least one reinforcing rib (36, 38) formed around the fluid inlet (14).

31. A ventilation device, comprising an expiratory valve (10) according to any of the preceding claims that is adapted to be connected to an expiration air conduit.

32. A method of assembling an expiratory valve (10) with a ventilation device (100), in which a valve membrane (20), with an abutment area formed along the outer periphery thereof, is brought into abutment with a top side (46) of a support (42) formed on a valve body (12) of the expiratory valve (10) and the expiratory valve (10) is mounted to the ventilation device (100), wherein the valve body (12) is deformed by clamping forces, wherein the surface (46) of the support (42) formed on the valve body (12) has an uneven design such that the deformation of the valve body (12) caused by clamping forces in mounting the expiratory valve (10) to the ventilation device (100) has the ef feet that the top side (46) of the support (42) becomes planar;
wherein the top side (46) of the support (42) is located in an outwardly curved imaginary surface with respect to a valve plenum (18) enclosed by the valve body (12).

## Revendications

1. Valve expiratoire (10) d'un appareil de ventilation mécanique assistée (100), présentant :
un corps de valve (12) dans lequel sont réalisés un orifice d'entrée pour fluide (14) et un orifice de sortie pour fluide (16) qui sont destinés à l'air respiratoire d'un patient ; dans laquelle le corps de valve (12) présente un support (42) qui comprend un côté supérieur (46) contre lequel peut venir s'appliquer une membrane de valve (20) qui comprend une surface d'appui qui est réalisée contre son bord extérieur ; dans laquelle le côté supérieur (46) du support (42) est réalisé de manière inégale ;
**caractérisée en ce que**
le côté supérieur (46) du support (42) est situé dans une surface virtuelle bombée vers l'extérieur par rapport à un plénum de valve (18) qui est entouré par le corps de valve (12), et l'inégalité du côté supérieur du support (42) est réalisée d'une manière telle que, ce n'est qu'après l'incorporation de la valve expiratoire (10) dans l'appareil de ventilation mécanique assistée (100) et qu'après la mise sous tension du corps de valve (12) d'une manière telle qu'il subit une déformation, que le côté supérieur (46) du support (42) vient se disposer en formant une surface plane, et que la membrane de valve (20) vient alors s'appuyer avec sa surface d'appui, sur toute l'étendue de sa surface, sur le côté supérieur (46) du support (42).

2. Valve expiratoire (10) selon la revendication 1, présentant en outre une membrane de valve (20) qui est attribuée à l'orifice d'entrée pour fluide (14), qui peut être déplacée au moins entre une position qui ferme l'orifice d'entrée pour fluide (14) et une position qui ouvre l'orifice d'entrée pour fluide (14) ; dans laquelle la membrane de valve (20) vient s'appuyer avec une surface d'appui qui est réalisée contre son bord externe, sur le côté supérieur du support (42).

3. Valve expiratoire (10) selon la revendication 2, présentant en outre un élément de serrage (44), en particulier une bague de serrage, qui coopère avec le corps de valve (12) d'une manière telle que l'élément de serrage (44) vient se disposer, à la périphérie externe de la membrane de valve (20) en engrènement de serrage avec la membrane de valve (20).

4. Valve expiratoire (10) selon l'une quelconque des revendications 1 à 3, dans laquelle le corps de valve (12) présente au moins une patte de montage (50) qui est réalisée pour entrer en engrènement avec un raccord de valve expiratoire d'un appareil de ventilation mécanique assistée (100).

5. Valve expiratoire (10) selon la revendication 4, dans laquelle le corps de valve (12) présente au moins deux pattes de montage (50) qui sont associées l'une à l'autre.

6. Valve expiratoire (10) selon l'une quelconque des revendications 1 à 5, dans laquelle le côté supérieur (46) du support (42) est disposé dans une surface imaginaire qui présente un bombement possédant une configuration de forme convexe.

7. Valve expiratoire (10) selon l'une quelconque des revendications 4 à 6, dans laquelle le côté supérieur (46) du support (42) s'incurve vers l'extérieur à partir de ladite au moins une patte de montage (50).

8. Valve expiratoire selon l'une quelconque des revendications 5 à 7, dans laquelle le bombement du support (42) s'étendant vers l'extérieur est maximal dans une zone qui est située entre les pattes de montage (50).

9. Valve expiratoire selon la revendication 8, dans laquelle le bombement du support (42) s'étendant vers l'extérieur est maximal le long d'une ligne qui s'étend entre les deux pattes de montage (50).

10. Valve expiratoire (10) selon l'une quelconque des revendications 6 à 9, dans laquelle la surface bombée imaginaire représente une surface latérale d'un cylindre.

11. Valve expiratoire (10) selon l'une quelconque des revendications 1 à 10, dans laquelle le corps de valve (12) présente une arête supérieure (48) qui est disposée dans une surface imaginaire qui s'incurve vers l'extérieur par rapport à un plénum de valve (18) qui est entouré par le corps de valve (12).

12. Valve expiratoire (10) selon la revendication 11, dans laquelle l'arête supérieure (48) du corps de valve (12) est disposée dans une surface imaginaire qui s'incurve vers l'extérieur en une configuration de forme convexe par rapport à un plénum de valve (18) qui est entouré par le corps de valve (12).

13. Valve expiratoire (10) selon la revendication 11 ou 12, dans laquelle l'arête supérieure (48) du corps de valve (12) s'incurve vers l'extérieur à partir de ladite au moins une patte de montage (50).

14. Valve expiratoire (10) selon l'une quelconque des revendications 11 à 13, dans laquelle le bombement de l'arête supérieure (48) du corps de valve (12) s'étendant vers l'extérieur est maximal dans une zone qui est située entre les pattes de montage (50).

15. Valve expiratoire (10) selon la revendication 14, dans laquelle le bombement de l'arête supérieure (48) du corps de valve (12) s'étendant vers l'extérieur est maximal le long d'une ligne qui s'étend entre les deux pattes de montage (50).

16. Valve expiratoire (10) selon l'une quelconque des revendications 12 à 15, dans laquelle la surface bombée imaginaire représente une surface latérale d'un cylindre.

17. Valve expiratoire (10) selon l'une quelconque des revendications 11 à 16, dans laquelle le côté supérieur (46) du support (42) et l'arête supérieure (48) du corps de valve (12) s'incurvent vers l'extérieur en étant orientés l'un vers l'autre.

18. Valve expiratoire (10) selon la revendication 17, dans laquelle le côté supérieur (46) du support (42) et l'arête supérieure (48) du corps de valve (12) sont disposés dans des surfaces imaginaires parallèles l'une à l'autre.

19. Valve expiratoire (10) selon l'une quelconque des revendications 3 à 18, dans laquelle la patte de montage (50) présente une surface d'engrènement (52) qui est destinée à venir s'engrener avec un élément d'engrènement antagoniste qui est prévu sur un appareil de ventilation mécanique assistée (100) ; dans laquelle la surface d'engrènement (52) est munie d'un chanfrein d'entrée (52a) qui est destiné au bombement du côté supérieur (46) du support (42) s'étendant vers l'extérieur.

20. Valve expiratoire (10) selon la revendication 19, dans laquelle la surface d'engrènement (52) présente une zone non chanfreinée (52b) qui fait suite au chanfrein d'entrée (52a).

21. Valve expiratoire (10) selon la revendication 19 ou 20, dans laquelle la surface d'engrènement (52) est munie d'une butée (54) à son extrémité.

22. Valve expiratoire (10) selon l'une quelconque des revendications 1 à 21, dans laquelle le corps de valve (12) est fabriqué à partir d'une matière plastique.

23. Valve expiratoire (10) selon la revendication 22, dans laquelle le corps de valve (12) représente un élément que l'on obtient par l'intermédiaire d'un moulage par injection.

24. Valve expiratoire (10) selon l'une quelconque des revendications 1 à 23, dans laquelle le corps de valve (12) forme un plénum de valve (18) qui possède une configuration en forme de pot, dans lequel débouche l'orifice d'entrée pour fluide (14).

25. Valve expiratoire (10) selon la revendication 24, dans laquelle l'orifice d'entrée pour fluide (14) est disposé dans une zone centrale du plénum de valve (18).

26. Valve expiratoire (10) selon la revendication 24 ou 25, dans laquelle le corps de valve (12) forme une base de valve (24) qui inclut au moins en partie le plénum de valve (18), base qui s'étend en partie entre l'orifice d'entrée pour fluide (14) et un bord externe du plénum de valve (18).

27. Valve expiratoire (10) selon la revendication 26, dans laquelle la base de valve (24) définit une voie d'écoulement pour fluide entre l'entrée pour fluide (14) et la sortie pour fluide (16), voie d'écoulement dont la section transversale s'élargit dans la direction de la sortie pour fluide (16).

28. Valve expiratoire (10) selon la revendication 26 ou 27, dans laquelle la base de valve (24) s'incurve vers l'extérieur dans la direction axiale dans une zone qui entoure l'orifice d'entrée pour fluide (14).

29. Valve expiratoire (10) selon la revendication 28, présentant en outre un bombement axial (32) qui s'étend vers l'extérieur, dont la profondeur augmente dans la direction de l'orifice de sortie pour fluide (16) autour de la périphérie de l'orifice d'entrée pour fluide (14).

30. Valve expiratoire (10) selon l'une quelconque des revendications 1 à 29, dans laquelle le corps de valve (12) présente au moins une nervure de renforcement (36, 38) qui est réalisée autour de l'orifice d'entrée pour fluide (14).

31. Appareil de ventilation mécanique assistée qui présente une valve expiratoire (10) selon l'une quelconque des revendications précédentes, qui peut venir se raccorder à une conduite destinée à l'air d'expiration.

32. Procédé pour le montage d'une valve expiratoire (10) à un appareil de ventilation mécanique assistée (100) ; dans lequel on met une membrane de valve (20) en contact avec une surface d'appui réalisée le long de sa périphérie externe, qui comprend un côté supérieur (46) d'un support (42) qui est réalisé contre un corps de valve (12) de la valve expiratoire (10), et on fixe la valve expiratoire (10) à l'appareil de ventilation mécanique assistée (100) ; dans lequel le corps de valve (12) subit une déformation sous l'influence de forces de serrage ; dans lequel la surface (46) du support (42) réalisé contre le corps de valve (12) est réalisé en présentant une inégalité d'une manière telle que, lors de la fixation de la valve expiratoire (10) à l'appareil de ventilation mécanique assistée (100), une déformation du corps de valve (12) qui résulte de forces de serrage fait en sorte que le côté supérieur (46) du support (42) se met à niveau ;
dans lequel le côté supérieur (46) du support (42) est disposé dans une surface imaginaire qui s'incurve vers l'extérieur par rapport à un plénum de valve (18) qui est entouré par le corps de valve (12).
